# EUROPEAN PATENT SPECIFICATION

(11) **EP 2 303 397 B1**
(45) Date of publication and mention of the grant of the patent: **23.04.2014**
(21) Application number: 09794854.1
(22) Date of filing: 14.05.2009
(51) Int. Cl.: A61N 1/05

(54) **STRAIN RELIEF IN AN IMPLANTABLE ELECTRODE ASSEMBLY**
SPANNUNGSENTLASTUNG BEI EINER IMPLANTIERBAREN ELEKTRODENANORDNUNG
RÉDUCTEUR DE TENSION INTÉGRÉ À UN ENSEMBLE ÉLECTRODES IMPLANTABLE

(30) Priority: 20.06.2008 AU 2008903143; 20.06.2008 AU 2008903144; 20.06.2008 AU 2008903145; 20.06.2008 AU 2008903146
(43) Date of publication of application: 06.04.2011
(73) Proprietor: Cochlear Limited, Macquarie University, NSW 2109 (AU)
(72) Inventor: DADD, Fysh, Macquarie University, NSW 2109 (AU); SCHULLER, Peter, Macquarie University, NSW 2109 (AU); PAWSEY, Nicholas Charles Kendal, Macquarie University, NSW 2109 (AU); MANOUCHEHRI, Shahram, Macquarie University, NSW 2109 (AU)
(74) Representative: Grünecker, Kinkeldey, Stockmair & Schwanhäusser
(86) International application number: PCT/US2009/043946
(87) International publication number: WO 2010/005627

(56) References cited:
- WO-A2-2006/097934
- US-A- 4 516 820
- US-A- 4 590 946
- US-A- 5 749 912
- US-A- 5 833 714
- US-A- 5 846 469
- US-A- 5 957 958
- US-A1- 2002 183 777
- US-B1- 6 178 355
- US-B1- 6 178 355
- US-B1- 6 208 882
- US-B1- 6 208 882
- US-B1- 6 671 553

## Description

### CROSS-REFERENCE TO RELATED APPLICATION

### BACKGROUND

### Field of the Invention

The present invention relates generally to implantable medical devices including an implantable electrode assembly, and more particularly, to strain relief in an implantable electrode assembly.

### Related Art

Hearing loss, which may be due to many different causes, is generally of two types, conductive and sensorineural. In some cases, a person suffers from hearing loss of both types. Conductive hearing loss occurs when the normal mechanical pathways for sound to reach the cochlea, and thus the sensory hair cells therein, are impeded, for example, by damage to the ossicles. Individuals who suffer from conductive hearing loss typically have some form of residual hearing because the hair cells in the cochlea are undamaged. As a result, individuals suffering from conductive hearing loss typically receive an acoustic hearing aid that generates mechanical motion of the cochlea fluid.

In many people who are profoundly deaf, however, the reason for their deafness is sensorineural hearing loss. Sensorineural hearing loss occurs when there is damage to the inner ear, or to the nerve pathways from the inner ear to the brain. As such, those suffering from some forms of sensorineural hearing loss are thus unable to derive suitable benefit from hearing prostheses that generate mechanical motion of the cochlea fluid. As a result, medical devices having one or more implantable components that deliver electrical stimulation signals to a patient or recipient ("recipient" herein) have been developed. Certain such implantable medical devices include an array of stimulating electrode contacts that deliver the stimulation signals to nerve cells of the recipient's auditory system, thereby providing the recipient with a hearing percept.

As used herein, the recipient's auditory system includes all sensory system components used to perceive a sound signal, such as hearing sensation receptors, neural pathways, including the auditory nerve and spiral ganglion, and parts of the brain used to sense sounds. Electrically-stimulating implantable medical devices include, for example, auditory brain stimulators and cochlear prostheses (commonly referred to as cochlear prosthetic devices, cochlear implants, cochlear devices, and the like; simply "cochlear implants" herein.)

Oftentimes sensorineural hearing loss is due to the absence or destruction of the cochlear hair cells which transduce acoustic signals into nerve impulses. It is for this purpose that cochlear implants have been developed. Cochlear implants provide a recipient with a hearing percept by delivering electrical stimulation signals directly to the auditory nerve cells, thereby bypassing absent or defective hair cells that normally transduce acoustic vibrations into neural activity. Such devices generally use an electrode array implanted in the cochlea so that the electrodes may differentially activate auditory neurons that normally encode differential pitches of sound.

Auditory brain stimulators are used to treat a smaller number of recipients with bilateral degeneration of the auditory nerve. For such recipients, the auditory brain stimulator provides stimulation of the cochlear nucleus in the brainstem.

Electrode assemblies that can be used in a human body according to the state-of-the-art are disclosed in US 6,208,882 B1 and US 6,178,355 B1. US 6,208,882 B1 relates to a stapedius reflex electrode and discloses all of the features in the preamble of claim 1. US 6,178,355 B1 relates to intracardiac defibrillation leads.

### SUMMARY

The present invention provides an implanted electrode assembly as claimed in claim 1.

In accordance with one aspect of the present invention, an implanted electrode assembly is provided. The electrode assembly comprises: an elongate carrier member; at least one electrode contact disposed in the carrier member; and at least one elongate conductive pathway disposed in the carrier member having a distal end attached to the at least one electrode contact and having a substantially planar strain relief formed therein that is located only in the distal region of the pathway.

In accordance with another aspect of the present invention, an implantable electrode assembly is provided. The electrode assembly comprises: an elongate carrier member; at least one electrode contact disposed in the carrier member; at least one elongate conductive pathway disposed in the carrier member having a distal end attached to the at least one electrode contact via a contact joint and having a strain relief formed therein; and an anchor arrangement securing the pathway to the electrode contact adjacent to the contact joint.

In accordance with a still other aspect of the present invention, an implantable electrode assembly is provided. The electrode assembly comprises: an elongate carrier member; at least one electrode contact disposed in the carrier member; at least one elongate conductive pathway disposed in the carrier member having a distal end attached to the at least one electrode contact; and an anchor arrangement securing the pathway to the electrode contact adjacent to the contact joint.

### BRIEF DESCRIPTION OF THE DRAWINGS

Aspects and embodiments of the present invention are described herein with reference to the accompanying drawings, in which:
FIG. 1 is a perspective view of an exemplary medical device, a cochlear implant having an electrode assembly in accordance with embodiments of the present invention;
FIG. 2 is a side view of an implantable component of a cochlear implant in which embodiments of the present invention may be advantageously implemented;
FIG. 3A is a cross-sectional view of a region of an electrode assembly, in accordance with embodiments of the present invention;
FIG. 3B is a cross-sectional view of a region of an electrode assembly, in accordance with embodiments of the present invention;
FIG. 4A is a top view of an conductive pathway having a strain relief formed in the distal region thereof, in accordance with embodiments of the present invention;
FIG. 4B is a side view of the conductive pathway of FIG. 4A attached to an electrode contact, in accordance with embodiments of the present invention;
FIG. 4C is a top view of the conductive pathway and attached electrode contact of FIG. 4B;
FIG. 5A is a top view of an electrode contact attached to an conductive pathway having an annular shaped strain relief formed therein, in accordance with embodiments of the present invention;
FIG. 5B is a top view of an electrode contact attached to an conductive pathway having a helical strain relief formed therein, in accordance with embodiments of the present invention;
FIG. 5C is a side view of an electrode contact attached to an conductive pathway having a helical strain relief formed therein, in accordance with embodiments of the present invention;
FIG. 5D is a perspective view of an electrode contact attached to an conductive pathway having a laterally extending strain relief formed therein, in accordance with embodiments of the present invention;
FIG. 5E is a top view of an electrode contact attached to a conductive pathway having a distally extending strain relief formed therein, in accordance with embodiments of the present invention;
FIG. 5F is a top view of an electrode contact attached to a conductive pathway having a distally extending strain relief formed therein, in accordance with embodiments of the present invention;
FIG. 6 is a cross-sectional view of a region of an electrode assembly, in accordance with embodiments of the present invention;
FIG. 7 is a top view of an electrode contact attached to two conductive pathways each having a strain relief feature therein, in accordance with embodiments of the present invention;
FIG. 8A is a side view of a conductive pathway secured to an electrode contact by an anchor arrangement, in accordance with embodiments of the present invention;
FIG. 8B is a side view of a conductive pathway secured to an electrode contact by an anchor arrangement, in accordance with embodiments of the present invention;
FIG. 8C is a side view of a conductive pathway secured to an electrode contact by an anchor arrangement, in accordance with embodiments of the present invention;
FIG. 8D is a side view of a conductive pathway secured to an electrode contact by an anchor arrangement, in accordance with embodiments of the present invention;
FIG. 9A is a flowchart illustrating a method of manufacturing an element of an electrode assembly, in accordance with embodiments of the present invention;
FIG. 9B is a flowchart illustrating a method of manufacturing an element of an electrode assembly, in accordance with embodiments of the present invention;
FIG. 9C is a flowchart illustrating a method of manufacturing an element of an electrode assembly, in accordance with embodiments of the present invention;
FIG. 9D is a flowchart illustrating a method of manufacturing an element of an electrode assembly, in accordance with embodiments of the present invention;
FIG. 9E is a flowchart illustrating a method of manufacturing an element of an electrode assembly, in accordance with embodiments of the present invention;
FIG. 9F is a flowchart illustrating a method of manufacturing an element of an electrode assembly, in accordance with embodiments of the present invention;
FIG. 9G is a flowchart illustrating a method of manufacturing an element of an electrode assembly, in accordance with embodiments of the present invention;
FIG. 10A is a flowchart illustrating a method of manufacturing elements of an electrode assembly, in accordance with embodiments of the present invention;
FIG. 10B is a flowchart illustrating a method of manufacturing elements of an electrode assembly, in accordance with embodiments of the present invention;
FIG. 11 is a flowchart illustrating a method of manufacturing an element of an electrode assembly, in accordance with embodiments of the present invention;
FIG. 12A is a perspective view of a strain relief forming tool in an open position, in accordance with embodiments of the present invention;
FIG. 12B is a perspective view of a strain relief forming tool in a closed position, in accordance with embodiments of the present invention; and
FIG. 12C is a perspective view of a strain relief forming tool in an open position, in accordance with embodiments of the present invention;
FIG. 12D is a perspective view of a strain relief forming tool in a closed position, in accordance with embodiments of the present invention;
FIG. 13 is a perspective view of a wire holding tool, in accordance with embodiments of the present invention; and
FIG. 14 is a block diagram illustrating tools for manufacturing an element of an electrode assembly, in accordance with embodiments of the present invention.

### DETAILED DESCRIPTION

Aspects of the present invention are generally directed to strain relief features in an implantable electrode assembly. More specifically, the electrode assembly includes at least one electrode contact and an elongate conductive pathway disposed in an elongate carrier member. and A distal end of the conductive pathway is attached to the at least one electrode contact via a contact joint, and has a strain relief formed therein. The strain relief decreases the susceptibility of the elongate conductive pathway and/or contact joint to breakage resulting from flexing/bending of the electrode assembly.

As described below, a strain relief in accordance with embodiments of the present invention may have a variety of arrangements. In certain embodiments, the strain relief is substantially planar and may be located, for example, only in a distal region of the conductive pathway. In other embodiments, the conductive pathway is secured to the electrode contact at a location adjacent to the contact joint to further protect the contact joint from breakage.

Embodiments are described herein primarily in connection with one type of implantable medical device, a hearing prosthesis, and more specifically a cochlear implant. Cochlear implants are hearing prostheses that deliver electrical stimulation, alone or in combination with other types of stimulation, to the cochlear of a recipient. Therefore, as used herein a cochlear implant refers to a device that delivers electrical stimulation in combination with other types of stimulation, such as acoustic and/or mechanical stimulation.

It would be appreciated that embodiments of the present invention may be implemented in any cochlear implant or other hearing prosthesis now know or later developed; including auditory brain stimulators (also known as auditory brainstem implants (ABIs)). Furthermore, it would be understood that embodiments of the present invention may be implemented in implantable medical devices other than cochlear implants such as neurostimulators, cardiac pacemakers/defibrillators, functional electrical stimulators (FES), spinal cord stimulators (SCS), *etc.*

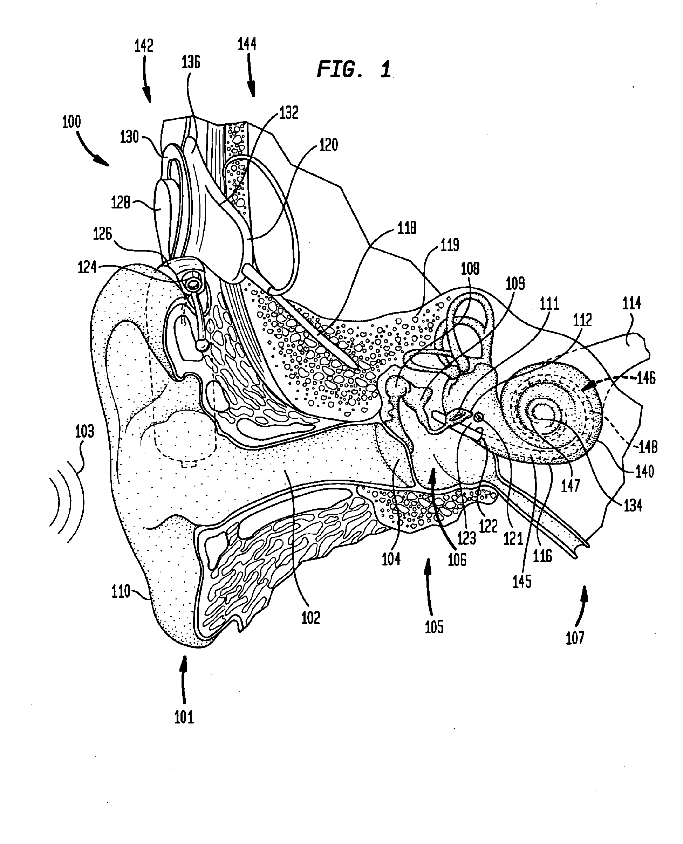

FIG. 1 is a perspective view of an exemplary cochlear implant 100 implanted in a recipient having an outer ear 101, a middle ear 105 and an inner ear 107. Components of outer ear 101, middle ear 105 and inner ear 107 are described below, followed by a description of cochlear implant 100.

In a fully functional ear, outer ear 101 comprises an auricle 110 and an ear canal 102. An acoustic pressure or sound wave 103 is collected by auricle 110 and channeled into and through ear canal 102. Disposed across the distal end of ear cannel 102 is a tympanic membrane 104 which vibrates in response to sound wave 103. This vibration is coupled to oval window or fenestra ovalis 112 through three bones of middle ear 105, collectively referred to as the ossicles 106 and comprising the malleus 108, the incus 109 and the stapes 111. Bones 108, 109 and 111 of middle ear 105 serve to filter and amplify sound wave 103, causing oval window 112 to articulate, or vibrate in response to vibration of tympanic membrane 104. This vibration sets up waves of fluid motion of the perilymph within cochlea 140. Such fluid motion, in turn, activates tiny hair cells (not shown) inside of cochlea 140. Activation of the hair cells causes appropriate nerve impulses to be generated and transferred through the spiral ganglion cells (not shown) and auditory nerve 114 to the brain (also not shown) where they are perceived as sound.

Cochlear implant 100 comprises an external component 142 which is directly or indirectly attached to the body of the recipient, and an internal or implantable component 144 which is temporarily or permanently implanted in the recipient. External component 142 typically comprises one or more sound input elements, such as microphone 124 for detecting sound, a sound processing unit 126, a power source (not shown), and an external transmitter unit 128. External transmitter unit 128 comprises an external coil 130 and, preferably, a magnet (not shown) secured directly or indirectly to external coil 130. Sound processing unit 126 processes the output of microphone 124 that is positioned, in the depicted embodiment, by auricle 110 of the recipient. Sound processing unit 126 generates encoded signals, sometimes referred to herein as encoded data signals, which are provided to external transmitter unit 128 via a cable (not shown).

Internal component 144 comprises an internal receiver unit 132, a stimulator unit 120, and an elongate stimulating lead assembly 118. Internal receiver unit 132 comprises an internal coil 136, and preferably, a magnet (also not shown) fixed relative to the internal coil. Internal receiver unit 132 and stimulator unit 120 are hermetically sealed within a biocompatible housing, sometimes collectively referred to as a stimulator/receiver unit. Internal coil 136 receives power and stimulation data from external coil 130, as noted above. Elongate stimulating lead assembly 118 has a proximal end connected to stimulator unit 120, and extends through mastoid bone 119. Lead assembly 118 has a distal region, referred to as electrode assembly 145, implanted in cochlea 140. As used herein the term "stimulating lead assembly," refers to any device capable of providing stimulation to a recipient, such as, for example, electrical or optical stimulation.

Electrode assembly 145 may be implanted at least in basal region 116 of cochlea 140, and sometimes further. For example, electrode assembly 145 may extend towards apical end of cochlea 140, referred to as cochlea apex 134. Electrode assembly 145 may be inserted into cochlea 140 via a cochleostomy 122, or through round window 121, oval window 112, and the promontory 123 or opening in an apical turn 147 of cochlea 140.

Electrode assembly 145 has disposed therein or thereon a longitudinally aligned and distally extending array 146 of electrode contacts 148, sometimes referred to as electrode array 146 herein. Throughout this description, the term "electrode array" means a collection of two or more electrode contacts, sometimes referred to simply as contacts herein. As would be appreciated, electrode array 146 may be disposed on electrode assembly 145. However, in most practical applications, electrode array 146 is integrated into electrode assembly 145. As used herein, electrode contacts or other elements disposed in a carrier refer to elements integrated in, or positioned on, the carrier member. As such, electrode array 146 is referred to herein as being disposed in electrode assembly 145. Stimulator unit 120 generates stimulation signals which are applied by electrodes 148 to cochlea 140, thereby stimulating auditory nerve 114.

In cochlear implant 100, external coil 130 transmits electrical signals (i.e., power and stimulation data) to internal coil 136 via a radio frequency (RF) link. Internal coil 136 is typically a wire antenna coil comprised of multiple turns of electrically insulated single-strand or multi-strand platinum or gold wire. The electrical insulation of internal coil 136 is provided by a flexible silicone molding (not shown). In use, implantable receiver unit 132 may be positioned in a recess of the temporal bone adjacent auricle 110 of the recipient.

As noted, FIG. 1 illustrates specific embodiments of the present invention in which cochlear implant 100 includes an external component 142. It would be appreciated that in alternative embodiments, cochlear implant 100 comprises a totally implantable prosthesis that is capable of operating, at least for a period of time, without the need of an external component. In such embodiments, all components of cochlear implant 100 are implantable, and the cochlear implant operates in conjunction with external component 142.

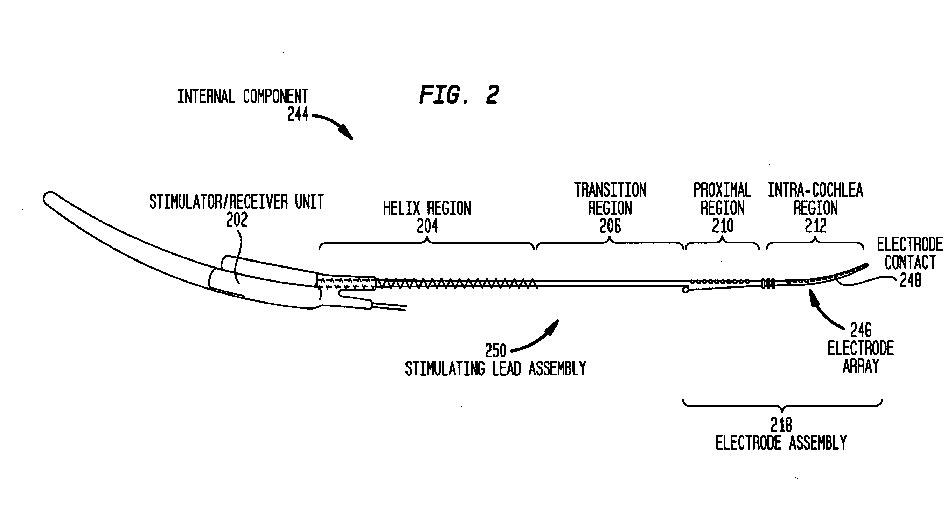

FIG. 2 is a simplified side view of an embodiment of internal component 144, referred to herein as internal component 244. As shown in FIG. 2, internal component 244 comprises a stimulator/receiver unit 202 which, as described above, receives encoded signals from an external component of the cochlear implant. Connected to stimulator/receiver unit 202 is a stimulating lead assembly 250. Stimulating lead assembly 250 terminates in an electrode assembly 218 that comprises a proximal region 210 and an intra-cochlear region 212. Intra-cochlear region 212 is configured to be implanted in the recipient's cochlea and has disposed thereon an array 246 of electrode contacts 248. Proximal region 210 is configured to be positioned outside of the recipient's cochlea.

In certain embodiments, electrode assembly 218 is configured to adopt a curved configuration during or after implantation into the recipient's cochlea. To achieve this, in certain embodiments, electrode assembly 218 is pre-curved to the same general curvature of a Cochlea. In such embodiments, electrode assembly 218 is referred to as perimodiolar electrode assembly that is held straight by, for example, a stiffening stylet (not shown) which is removed during implantation so that the electrode assembly may adopt its curved configuration when in the cochlea. Other methods of implantation, as well as other electrode assemblies which adopt a curved configuration, may be used in embodiments of the present invention.

In other embodiments, electrode assembly 218 is a non-perimodiolar electrode assembly which does not adopt a curved configuration. For example, electrode assembly 218 may comprise a straight electrode assembly or a mid-scala assembly which assumes a mid-scala position during or following implantation.

In the illustrative embodiments of FIG. 2, stimulating lead assembly 250 further comprises a helix region 204 and a transition region 206 connecting stimulator/receiver unit 202 to electrode assembly 218. Helix region 204 prevents the connection between stimulator/receiver 202 and electrode assembly 218 from being damaged due to movement of internal component 244 which may occur, for example, during mastication.

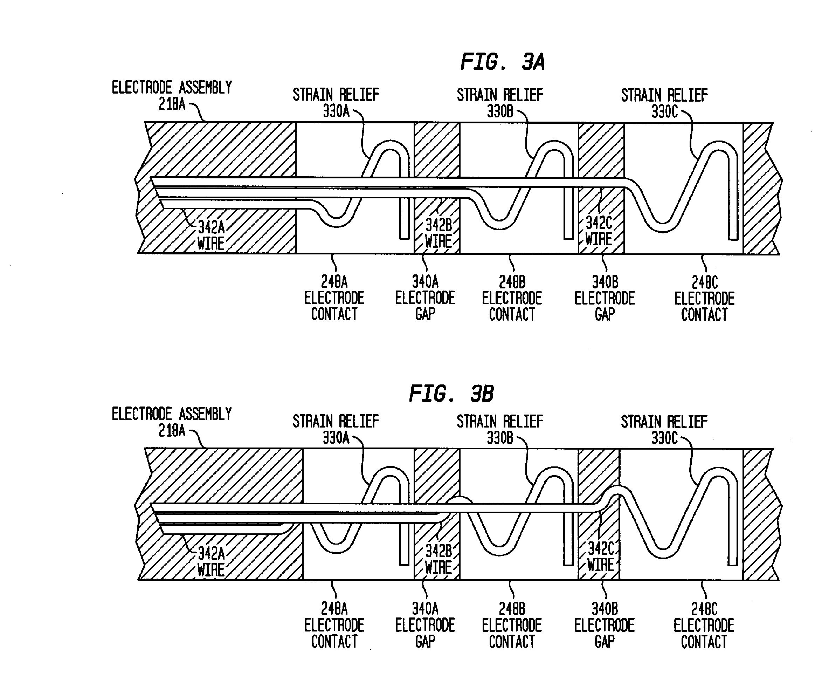

As noted above, aspects of the present invention are generally directed to strain relief features in an implantable electrode assembly. FIGS. 3A and 3B are top-views of portions of electrode assembly 218 including strain relief features in accordance with certain embodiments of the present invention. The portion of electrode assembly 218 shown in FIGS. 3A and 3B comprise three electrode contacts 248. Electrode contacts 248 are preferably made from platinum, but any other suitable material such as Iridium, a platinum/iridium alloy, or other platinum or iridium alloy may be used, as will be understood by a person skilled in the art. Furthermore, FIGS. 3A and 3B illustrate electrode contacts 248 which are substantially planar and have a rectangular shape. However, it should be appreciated that electrode contacts 248 may have other shapes, such as, for example, a U-shape, square, circular, oval, *etc.*

As shown in FIGS. 3A and 3B, each electrode contact 248 has a conductive pathway 342 which extends from the electrode contacts to stimulator/receiver unit 202 (FIG. 2). Conductive pathways 342 are shown as wires 342 in FIGS. 3A and 3B. However, it should be appreciated that in accordance with embodiments of the present invention, conductive pathways 342 may comprise, for example, multi-strand wires or may comprise cut, punched, or machined foil strips of platinum or other material.

As noted above, electrical stimulation signals are generated by stimulator unit 202 and provided to electrode contacts 248 via wires 342. Electrode contacts 248 deliver the electrical stimulation signals to the recipient. Thus, to ensure proper operation of the cochlear implant, it is important to maintain the electrical connection between stimulator unit 202 and electrode contacts 248. Embodiments of the present invention are configured to maintain the electrical connection by providing a strain relief feature, or simply strain relief herein, in wires 342. As used herein, a strain relief refers to a non-linear section of the wire that is embedded in a flexible material, such as silicone.

During use, the flexible material holds the strain relief in shape, but allows movement of the stored length of with when loads are placed on wires 342 as a result of bending and straightening of electrode assembly 218. The strain reliefs prevent breakage of the electrical connection between stimulator unit 202 and electrode contacts 248 in response to such flexing/bending of electrode assembly 218A.

In the illustrative embodiments of FIGS. 3A and 3B, the strain relief features are referred to as strain reliefs 330 and 332, respectively. Each strain relief 330, 332 of FIGS. 3A and 3B are located only in the distal region of elongate wires 342. In other words, strain reliefs 330, 332 are entirely localized to regions that are proximate to an electrode contact, referred to as an electrode contact region herein.

Localization of strain reliefs 330, 332 to the electrode contact region helps to protect the portions of the electrical connection between stimulator/receiver unit 202 and electrode contacts 248 that are most susceptible to breakage. The region of the electrical connection between stimulator/receiver unit 202 and electrode contacts 248 disposed in the electrode contact region is susceptible to breakage for several reasons. As discussed below, several methods, including resistance welding, wire bonding, and crimping may be used to join conductive pathways 342 to electrode contacts 248. Alternatively, conductive pathways 342 and electrode contacts may be cut, stamped or fabricated (i.e. thin film fabrication) from a single piece of material. All of the formed connections are themselves susceptible to breakage, but the above methods also potentially damage the conductive pathway making the pathway more prone to breakage. For instance, resistance welding or wire bonding may result in a heat affected zone (HAZ) and deformation to the wire, while crimping may result in stress imparted weakness (e.g. from deformation of the material). Also, cutting or stamping may result in a geometric weakness in the connecting region, while thin film fabrication is prone to surface tension effects. Therefore, for these and other regions, the electrode contact region is particularly prone to damage during use.

In the embodiments of FIG. 3A, each strain relief 330 is localized to the electrode contact region so that the strain relief is positioned entirely alongside an electrode contact 248, and the length of each strain relief is less than or equal to the length of the electrode contact. The length of each strain relief 330 and electrode contact 248 refers to the distance from the most proximal end of the relief or contact to the most distal end of the relief or contact.

In the embodiments of FIG. 3B, each strain relief 332 is localized to the electrode contact region so that the strain relief is positioned alongside an electrode contact 248. In these embodiments, the length of each strain relief is less than or equal to the length of the electrode contact and the length of the region between the electrode contact and the next most proximal electrode contact, referred to as electrode gap 340. The length of each strain relief 332, electrode contact 248, or electrode gap 340 refers to the distance from the most proximal end of the relief, contact or gap to the most distal end of the relief, contact or gap.

Thus, the illustrative embodiments of FIGS. 3A and 3B optimize the length of the strain relief by limiting it to the region in which the electrical connection between the stimulator unit and the electrode contact is most likely to be broken as a result of shock, misuse, flexing of the electrode assembly, or other events or circumstances which result in the application of stress to the weld. Positioning the strain reliefs proximate to the electrode contacts 248 has several advantages. First, because each strain relief is proximate to an electrode contact, the strain relief is formed in the distal region of the wire. As described below, a wire with such a distally positioned strain relief is relatively easy to handle during manufacture of an electrode assembly.

A second advantage of strain reliefs 330, 332 positioned proximate to electrode contacts 248, is that the strain reliefs do not overlap with one another. The non-overlapping strain reliefs created a compact wire bundle, and thus saves space within electrode assembly 218. Furthermore, because the strain reliefs to not overlap, the proximal portions of the wires are in a close bundle. The close bundle helps to protect the wires from breakage.

In alternative embodiments of the present, the strain reliefs are positioned proximate to electrode contacts 248, but extend beyond the next most proximal electrode contact. In certain such embodiments, the strain reliefs extend the length of intra-cochlea region 212.

As detailed above, FIGS. 3A and 3B illustrate embodiments of the present invention in which strain reliefs 330, 332 are positioned in specific physical locations. It should be appreciated that other physical locations for a strain relief are within the scope of the present invention, and the illustrative embodiments of FIGS. 3A and 3B should not be construed to limit the available locations. For instance, in accordance with other embodiments of the present invention, a conductive pathway may be attached to a first electrode contact, and a strain relief may be formed therein alongside the next most proximal electrode contact and/or next proximal electrode contact and adjoining electrode gap.

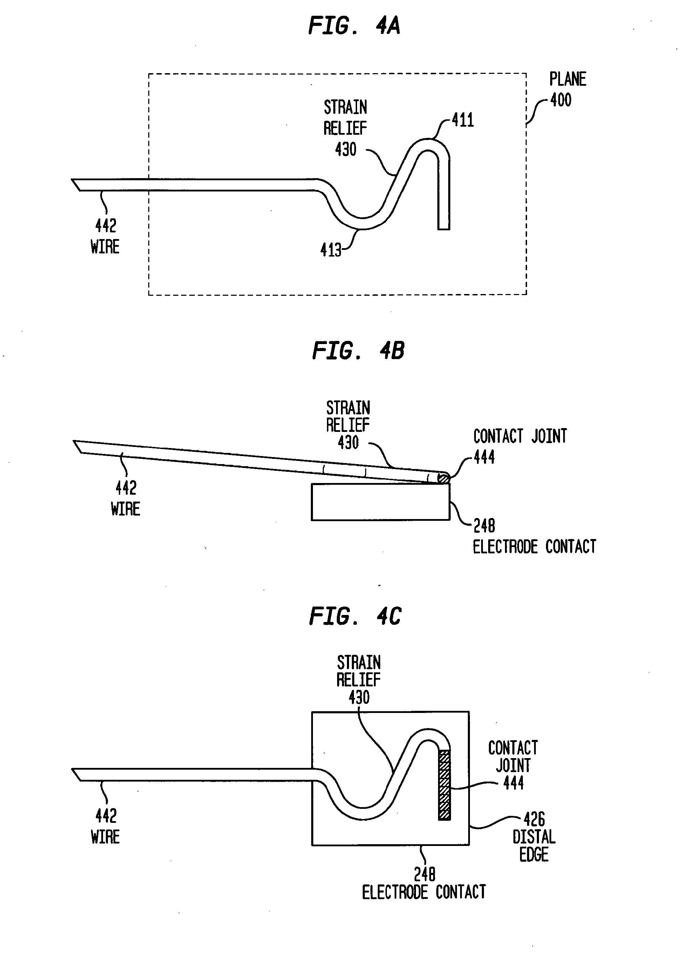

FIGS. 4A and 4B illustrate a wire 442 having a strain relief 430 formed therein. FIG. 4A is a schematic top view of wire 442, while FIG. 4B is a schematic side view of wire 442. In the embodiments of FIGS. 4A and 4B, strain relief 430 is a planar strain relief. That is, strain relief 430 is substantially two dimensional and is positioned substantially within a single plane 400 (excluding wire thickness).

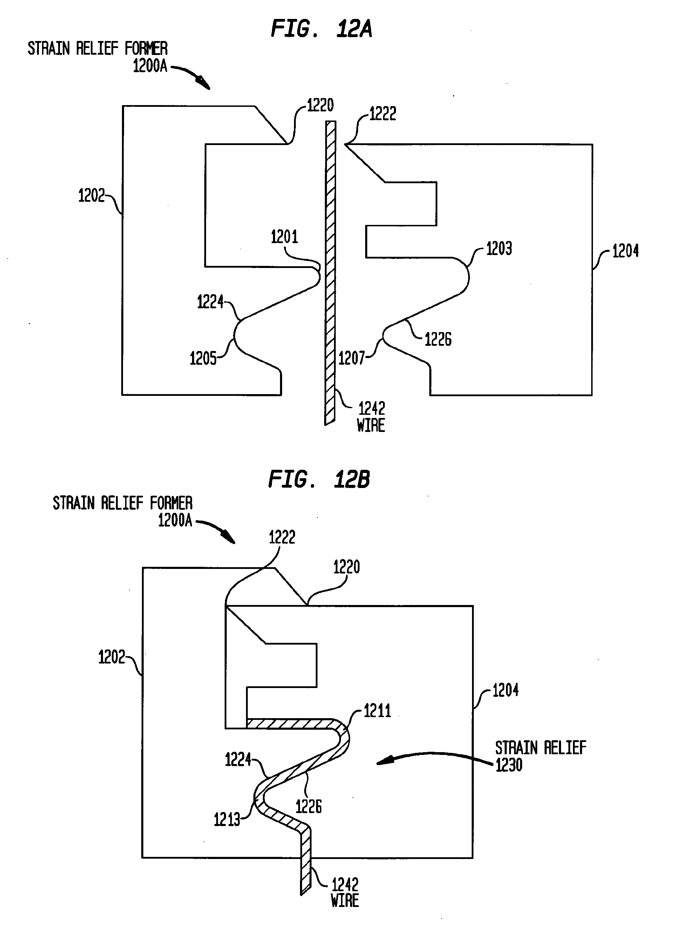

Strain relief 430 illustrated in FIGS. 4A-4C comprises a non-linear section of wire 442. Specifically, strain relief 430 comprises a full turn 411 of wire 442, and a half turn 413 of the wire. As shown in FIG. 4A, a full turn of wire or conductive pathway refers to a section of wire/pathway that travels a first direction for a first distance, has a general U-shaped portion, and which extends away from the U-shape for a second distance which is approximately the same as the first distance. In contrast, a half turn refers to a section of wire that only comprises a U-shaped potion. As detailed below with reference to FIGS. 12A-12D, in embodiments of the present invention the turns of a strain relief, such as strain 430, are asymmetric in size and are referred to as asymmetric turns of wire/pathway. In some embodiments, each distally positioned turn is larger than the next most proximal turn. In other embodiments, each distally positioned turn is smaller than the next most proximal turn. The size of a turn refers to the change in length of wire over the original longitudinal distance of the wire. It should be appreciated that an increase in size increases the largest dimensions of the turn perpendicular to the longitudinal axis of wire 442.

As shown in FIG. 4B, planar strain relief 430 is formed in a distal region of wire 442. That is, planar strain relief 430 is formed at or near the distal end of wire 442, and is positioned alongside an electrode contact 248. As described in detail below, wire 442 is attached to electrode contact 248 at contact joint 444.

As noted above, a variety of methods may be implemented to attach or join a conductive pathway, such as a wire, to an electrode contact. The attachment or connection point between a wire or other conductive pathway and an electrode contact is referred to herein as contact joint 444. Contact joint 444 may be provided by resistance welding, wire bonding, crimping, laser welding, etc. In certain circumstances, the wire and electrode contact are cut out or stamped from a single sheet of metal, and the contact joint is the region where the element changes from the electrode shape to the wire shape.

As noted above, a strain relief, such as strain relief 430, comprises a non-linear section of wire 442 that is surrounded by a flexible material such as silicone. In order to provide movement of the strain relief 430 in response to, for example, flexing/bending of the electrode assembly, strain relief 430 must be spaced a distance from electrode contact 248 so that the strain relief is not fixed to a rigid structure, such as the electrode contact. This distance may comprise, for example, 50 to 100 microns.

The desired distance between electrode contact 248 and strain relief 430 may be provided in several different manners. For instance, as shown in FIG. 4B, wire 442 may be presented to the electrode contact at an angle. This angle with respect to electrode contact 248 refers to the face that the distal end of wire 442 are in closer proximity to electrode contact 248 than strain relief 430. In other embodiments, the desired spaced is obtained by locating strain relief 430 between electrode contacts, rather than beside an electrode contact as shown in FIG. 4B.

FIG. 4C is a top view of wire 442 attached to electrode contact 248 via contact joint 444. As shown, in order to maximize the area of electrode contact 248 that strain relief 430 may be positioned alongside, contact joint 444 is formed parallel to distal edge 426 of electrode contact 248. Furthermore, contact joint 444 is formed as close as practicable to edge 426. For example, in some practical applications contact joint 444 may be spaced approximately 25 microns.

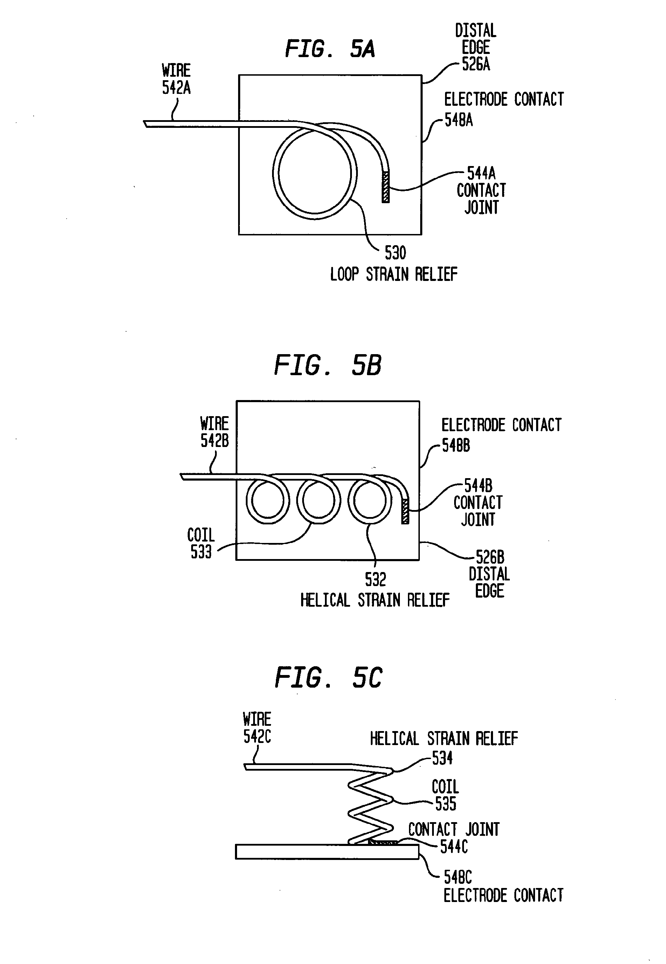

FIG. 5A is a schematic top view of an electrode contact 548A attached to a wire 542A having a strain relief 530 formed therein. As shown, strain relief 530 has a substantially annular shape and comprises a single coil of wire 542A. As such, strain relief 530 is referred to as a loop strain relief 530.

In the embodiments of FIG. 5A, loop strain relief 530 is shown formed in the distal region of wire 542A and is alongside electrode contact 548A. That is, the length of the strain relief is less than or equal to the length of the electrode contact. The length of loop strain relief 530 and electrode contact 548A refers to the distance from the most proximal end of the relief or contact to the most distal end of the relief or contact. It should be appreciate that in alternative embodiments, loop strain relief 530 may be formed in other locations of wire 542A.

As shown, the distal end of wire 542A is attached to electrode contact 548A via a contact joint 544A. In the embodiments of FIG. 5A, contact joint 544A is formed parallel to distal edge 526A of electrode contact 548A. Furthermore, contact joint 544A is formed as close as practicable to edge 526A.

FIG. 5B is a schematic top view of an electrode contact 548B attached to a wire 542B having a strain relief 532 formed therein. As shown, strain relief 532 comprises a plurality of loops or coils 533 of wire 542B and is referred to as helical strain relief 532. In certain embodiments of FIG. 5B, coils 533 of helical strain relief 532 are substantially positioned in a single plane which is alongside electrode contact 548B. In other embodiments, coils 533 extend about an elongate axis which is parallel to, or perpendicular, the proximal end of wire 542B.

Furthermore, in the embodiments of FIG. 5B, helical strain relief 532 is shown formed near the distal end of wire 542B and is positioned proximate to electrode contact 548A. Specifically, helical strain relief 532 is positioned alongside electrode contact 548B, and the length of the strain relief is less than or equal to the length of the electrode contact. The length of helical strain relief 532 and electrode contact 548B refers to the distance from the most proximal end of the relief or contact to the most distal end of the relief or contact.

In alternative embodiments, helical strain relief 532 has a length that is greater than electrode contact 548B. For exampled, helical strain relief 532 may extend to the next most proximal electrode and is positioned within electrode contact 548A and the adjacent electrode gap, as described above with reference to FIG. 3B. In other embodiments, helical strain relief 532 may be formed in wire 542B so that the strain relief extends the length of an intra-cochlea region of an electrode assembly that is formed using the wire. It should also be appreciated that helical strain relief 532 may be formed in other locations of wire 542B than that shown.

As shown, the distal end of wire 542B is attached to electrode contact 548B via a contact joint 544B. In the embodiments of FIG. 5B, contact joint 544B is formed parallel to distal edge 526B of electrode contact 548B. Furthermore, contact joint 544B is formed as close as practicable to edge 526B.

FIG. 5C is a schematic side view of an electrode contact 548C attached to a wire 542C having a strain relief 534 formed therein. As shown, strain relief 534 comprises a plurality of loops or coils 535 of wire 542C and is referred to as helical strain relief 534. In certain embodiments of FIG. 5C, coils 535 of helical strain relief 534 extend about an elongate axis which is perpendicular to the remainder of wire 542C.

Furthermore, in the embodiments of FIG. 5C, helical strain relief 534 is shown formed near the distal end of wire 542C and is positioned proximate to electrode contact 548A. Specifically, helical strain relief 534 is positioned alongside electrode contact 548C, and the length of the strain relief is less than or equal to the length of the electrode contact. The length of helical strain relief 534 and electrode contact 548C refers to the distance from the most proximal end of the relief or contact to the most distal end of the relief or contact.

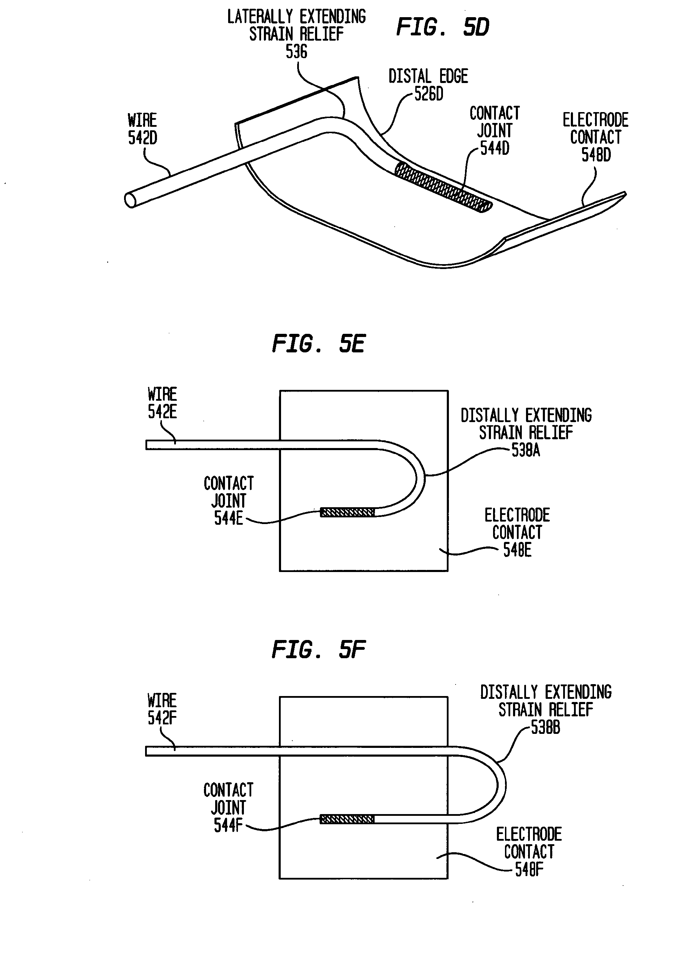

FIG. 5D is a schematic top view of an electrode contact 548D attached to a wire 542D having a strain relief 536 formed therein. In the embodiments of FIG. 5D, electrode contact 548D has a U-shape. Strain relief 536 extends laterally from contact joint 544D up a side of the U-shape. As such, strain relief 536 is referred to as a laterally extending strain relief 536.

As shown, the distal end of wire 542D is attached to electrode contact 548D via a contact joint 544D. In the embodiments of FIG. 5D, contact joint 544D is formed parallel to distal edge 526D of electrode contact 548D. Furthermore, contact joint 544D is formed as close as practicable to edge 526D.

FIGS. 5E is a schematic top view of an electrode contact 548E attached to a wire 542E having a strain relief 538A formed therein. In the embodiments of FIG. 5E, strain relief 538A extends distally from the attachment to electrode contact 548E, and is referred to as distally extending strain relief 538A.

FIG. 5F is a schematic top view of an electrode contact 548F attached to a wire 542F having a distally extending strain relief 538B formed therein. Distally extending strain relief 538B is similar to strain relief 538A of FIG. 5E and extends distally from the attachment to electrode contact 548F. In the embodiments of FIG. 5F, at least a portion of distally extending strain relief 538B extends past the distal end of electrode contact 548F.

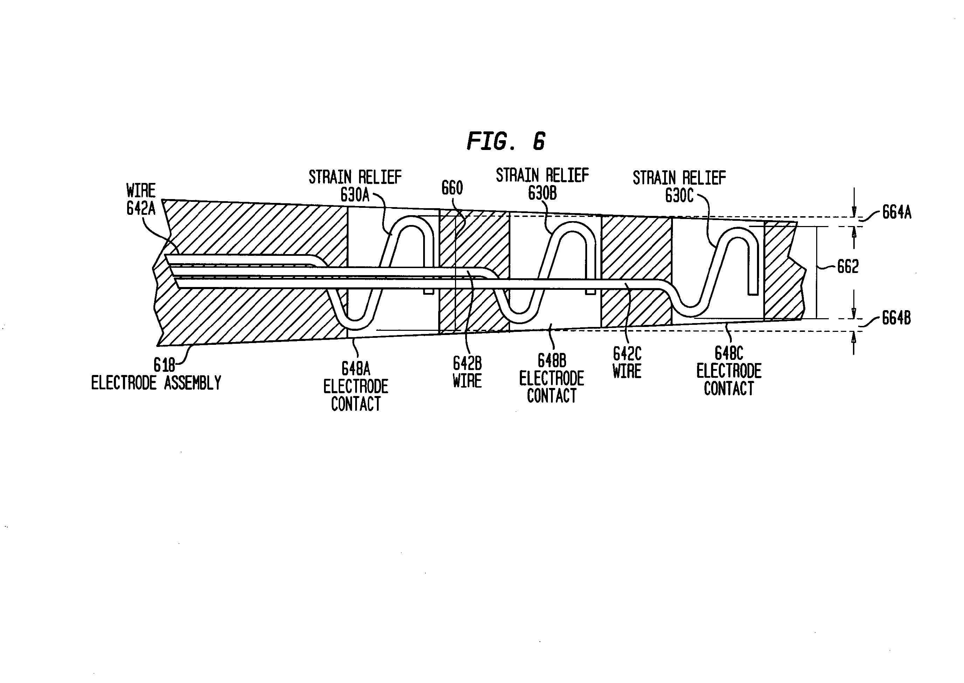

As is well known in the art, an electrode assembly configured for implantation in a cochlea has a diameter that decreases towards the distal end of the electrode assembly. That is, the width of electrode assembly tapers to a distal tip. In certain embodiments of the present invention, strain reliefs are formed to take advantage of the changing diameter of an electrode assembly. Specifically, wires within the electrode assembly have different shaped and/or sized strain reliefs based on the size of the electrode assembly in the region of the strain relief. In other words, the wires include different strain reliefs designed to maximize the use of the electrode assembly. FIG. 6 is a schematic top-view of a region of a tapered electrode assembly 618 formed in accordance with such embodiments of the present invention. As would be appreciated, the taper of electrode assembly 618 shown in FIG. 6 is exaggerated to illustrate the embodiments of the present invention. In some practical applications, an electrode assembly in accordance with embodiments of the present invention may taper approximately 0.3 mm over a length of 10 mm. That is, the diameter of the electrode assembly reduces 0.3 mm over a 10 mm span.

As shown, a first strain relief 630A is formed in a wire 642A. The distal end of wire 642A is attached to electrode contact 648A. Strain relief 630A has a width 660 which is configured to maximize the space available in the region of electrode assembly 618 in which electrode contact 648A is positioned. As noted above, the length of a strain relief refers to the distance from the most proximal end of the relief to the most distal end of the relief. As used herein, the width of a strain relief refers to the largest dimensions of the strain relief in the direction perpendicular to the length of the strain relief.

Electrode assembly 618 further includes a second strain relief 630C formed in a wire 642C. The distal end of wire 642C is attached to electrode contact 648C. Strain relief 630C has a width 662. The width 662 of strain relief 630C is smaller than the width of strain relief 630A, shown by arrows 664. Again, the width 662 of strain relief 630C is selected to maximize the space available in the region of electrode assembly 618 in which electrode contact 648C is positioned. As would be appreciated, the difference in width between strain reliefs 630A, 630C is due to the taper of electrode assembly 618.

Electrode assembly 618 further includes a third strain relief 630B which is optimized for the space available at electrode contact 648B. For ease of illustration, the decrease in size between strain reliefs 630A and 630B are not shown herein.

The embodiments of FIG. 6 have been illustrated with respect to three electrode contacts 648 within electrode assembly 618. It would be appreciated that additional electrode contacts may be provided and that each electrode contact may be attached to a wire having strain reliefs of various widths.

Furthermore, the embodiments of FIG. 6 have been illustrated with respect to strain reliefs 630 having the same non-linear shape. It should be appreciated that in alternative embodiments, different non-linear patterns may be selected based on width of the electrode assembly.

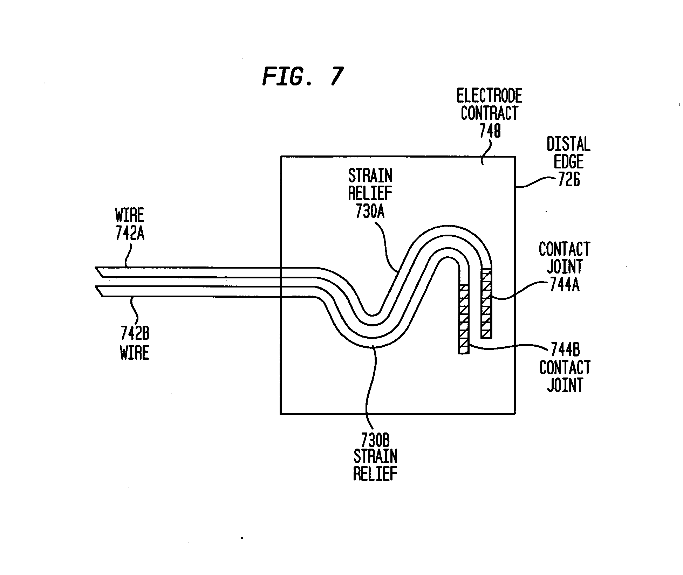

As noted above, embodiments of the present invention are directed to maintaining the electrical connection between stimulator/receiver unit 202 (FIG. 2) and an electrode contact by providing a strain relief in the wires electrically connecting the stimulator unit to the electrode contact. FIG. 7 illustrates a further embodiment of the present invention in which two wires 742A, 742B are attached to an electrode contact 748 and extend to a stimulator unit. As shown, each wire 742A, 742B, has formed therein a strain relief 730A, 730B, respectively. Furthermore, the distal ends of wires 742A and 742B are attached to electrode contact 748 via contact joints 744A, 744B, respectively. In the embodiments of FIG. 7, contact joints 744 are each formed parallel to distal edge 726 of electrode contact 748. Furthermore, contact joints 744 are each formed as close as practicable to edge 726.

As detailed above, strain reliefs 730 are configured to reduce the susceptibility of the electrical connection between the stimulator unit and electrode contact 748 to breakage as a result of, for example, flexing/bending of an electrode assembly in which the electrode contact and wires 742 are positioned. The use of two wires 742 extending between electrode contact 748 and the stimulator unit further reduces the susceptibility of the electrical connection to breakage by providing a redundant path between the stimulator unit and the electrode contact. If one of wires 742 is broken, the electrical connection is maintained by the second wire.

As noted above, a variety of methods may be implemented to attach or join a conductive pathway, such as a wire, to an electrode contact. The attachment or connection point between a wire or other conductive pathway and an electrode contact is referred to herein as a contact joint. A contact joint may be provided by welding, wire bonding, crimping, laser welding, *etc.* In certain circumstances, the wire and electrode contact are cut out or stamped from a single sheet of metal, and the contact joint is the region where the element changes from the electrode shape to the wire shape.

As noted above, a conductive pathway is attached to an electrode contact via a contact joint. Methods for forming this contact joint including resistance welding, wire bonding, and crimping. Alternatively, conductive pathways and electrode contacts may be cut, stamped or fabricated (i.e. thin film fabrication) from a single piece of material. All of the formed connections are themselves susceptible to breakage, but the above methods also potentially damage the conductive pathway making the pathway more prone to breakage. For instance, resistance welding or wire bonding may result in a heat affected zone (HAZ) and deformation to the wire, while crimping may result in stress imparted weakness (e.g. from deformation of the material). Also, cutting or stamping may result in a geometric weakness in the connecting region, while thin film fabrication is prone to surface tension effects. Therefore, for these and other regions, the electrode contact region is particularly prone to damage during use.

Also as noted, as a result of, for example, flexing/bending of an electrode assembly, a wire connecting an electrode contact to a stimulator unit is exposed to forces that may damage the electrical connection between the stimulator unit and the electrode contact. Thus, a strain relief is provided to reduce the likely that the wire will break as a result of such flexing/bending. Embodiments of the present invention provide additional features which further serve to maintain the integrity of the electrical connection by protecting the contact joint from external forces. In particular, embodiments of the present invention are configured to protect the contact joint from flexing/bending forces by securing the conductive pathway to the electrode contact adjacent to the contact joint with an anchor arrangement. As used herein, an anchor arrangement refers to a collection one or more elements positioned substantially between the strain relief and the contact joint that are used to secure the section of wire to the electrode contact.

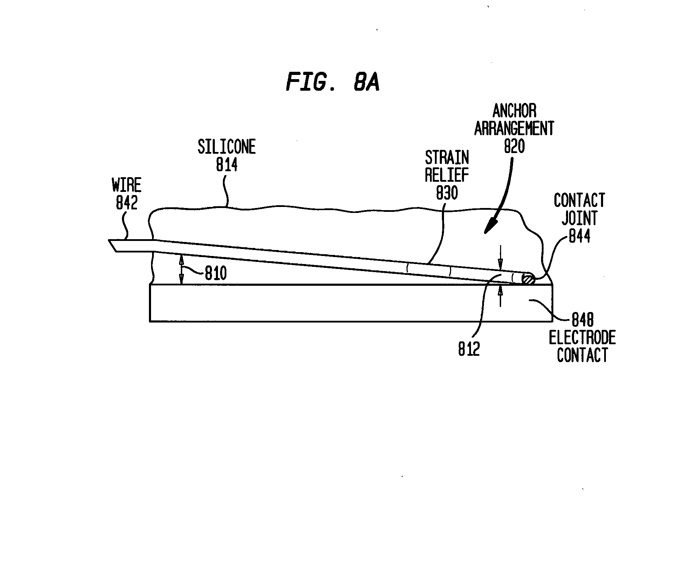

FIG. 8A is a schematic side view of an exemplary anchor arrangement 820 in accordance with embodiments of the present invention. As shown, wire 842 is attached to an electrode contact 848 by a contact joint 844. In the embodiments of FIG. 8A, wire 842 has a strain relief 830 formed therein. The distal end of wire 842 is positioned alongside electrode contact 848 and extends from electrode contact 848 at angle. The angle may be, for example, approximately fifteen degrees.

In the embodiments of FIG. 8A, due to the angle at which wire 842 extends from contact joint 844, the wire is spaced a first distance 812 from electrode contact 848 adjacent to contact joint 844, but wire 842 is spaced a second larger distance 810 from the electrode contact farther away from contact joint 844. As shown, a material 814, is used to fill a portion of the space between wire 842 and electrode contact 848, and may extend over wire 842. The material is configured to adhere the wire 842 to the electrode contact. The material may comprise silicone or silicone adhesive, and is shown in FIG. 8A as silicone 814.

Due to the close proximity of wire 842 and contact 848 (distance 812) adjacent to contact joint 844, silicone 814 substantially prevents movement of the portion of wire 848 that is adjacent to contact, joint 844, and thus helps to prevent damage to the contact joint as a result of bending of the electrode assembly.

In contrast, as the distance between wire 842 and electrode contact 848 increases, more silicone 814 is positioned there between. This increasing larger amount of silicone between wire 842 and electrode contact 848 provides for increasing greater freedom of movement of wire 842. Thus, in the embodiments of FIG. 8A, a significant portion of strain relief 830 is positioned spaced from electrode contact 848 so as to permit the desired movement of the strain relief in response to bending of the electrode assembly as discussed above.

The embodiments of FIG. 8A illustrate an anchor arrangement 820 in which the space between wire 842 and electrode contact 848 is filled with a silicone or silicone adhesive. In other embodiments, a different material such as silicone rubber or medical grade epoxy may be used to fill a portion of the space between wire 842 and electrode contact 848.

In certain embodiments of the present invention, the same flexible material, such as silicone, may be used to form the anchor arrangement and to surround the strain relief. However, it would be appreciated that materials such as epoxy cannot be used to surround the strain relief because such materials do not permit sufficient movement of the strain relief upon bending/flexing of the electrode assembly.

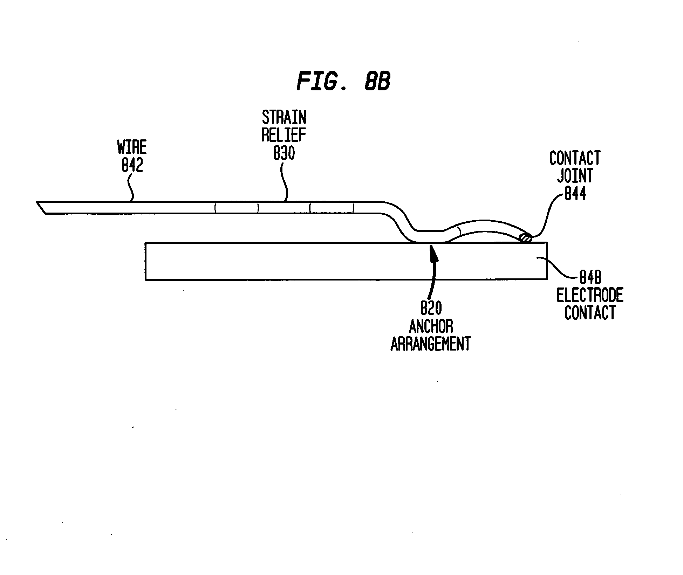

FIG. 8B is a schematic side view of alternative anchor arrangement 822 in accordance with embodiments of the present invention. Similar to the embodiments detailed above with reference to FIG. 8A, a wire 842 is attached to an electrode contact 848 by a contact joint 844. In the embodiments of FIG. 8B, wire 842 has a strain relief 830 formed therein. The distal end of wire 842 is positioned alongside electrode contact 848.

In the illustrative embodiments of FIG. 8B, anchor arrangement 822 comprises an added fixation element which directly secures wire 842 adjacent to electrode contact 848. This fixation element may comprise a weld that secures wire 842 to electrode contact 848. Thus, in certain embodiments of FIG. 8B, a weld is used as contact joint 844, and as anchor arrangement 822. In these embodiments, the added fixation element is configured to be weaker than the wire 842. That is, in the event of a stress event or the repeated bending of an electrode assembly in which the electrode contact and wire are positioned, the anchor arrangement is configured to break prior to breakage of wire 842. This helps to ensure that the electrical connection between the stimulator unit and the electrode contact is maintained.

As would be appreciated, relatively strong welds, such as a fusion welds, are used to attach the distal end of a conductive pathway to an electrode contact. In embodiments in which a weld is used as an anchor arrangement, the anchoring weld is configured to break prior to breakage of the wire. Furthermore, it is desirable to damage the wire as little as possible during the anchoring processed. Therefore, an anchoring weld comprises a tack weld rather than a fusion weld. A tack weld is weaker than a fusion weld, and the tack welding process helps to reduce heat affected zones in the wire caused by the welding process.

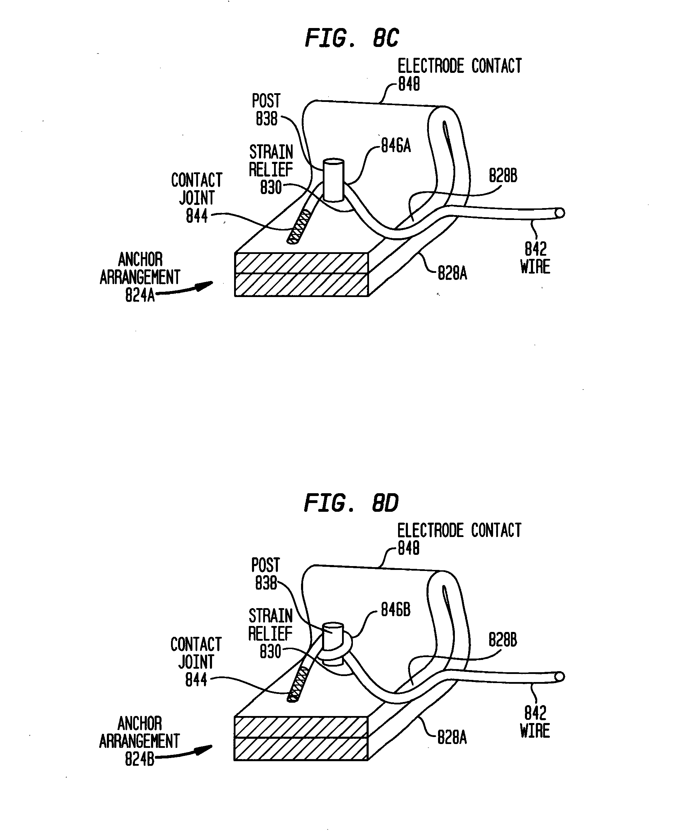

FIGS. 8C and 8D are cross-sectional perspective views of alternative anchor arrangements 824 in accordance with embodiments of the present invention. Similar to the embodiments detailed above with reference to FIGS. 8A and 8B, a wire 842 is attached to an electrode contact 848 by a contact joint 844. In the embodiments of FIGS. 8C and 8D, contact joint 844 comprises a weld, and wire 842 has a strain relief 830 formed therein.

In the embodiments of FIGS. 8C and 8D, anchor arrangements 824 comprise a section 846 of wire 842 that is at least partially wound or looped around a mechanical feature of electrode contact 848. As shown, electrode contact 848 comprises a U-shape and has a post 838 on the face thereof. A section 846 of wire 842 is wound around post 838 between strain relief 830 and contact joint 844. In the embodiments of FIG. 8D, section 846B comprises at least one full loop of wire wound around post 838, while in the embodiments of FIG. 8C section 846A is only partially wound around post 838.

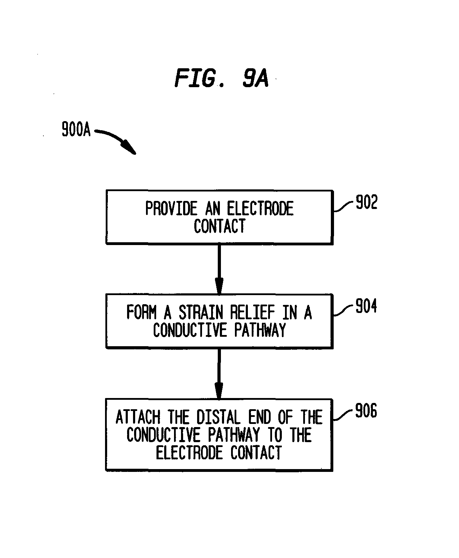

As noted above, an electrode assembly in accordance with embodiments of the present invention includes several elements, such as a carrier member, one or more electrode contacts, and conductive pathways extending from each of the one or more electrode contacts which electrically connect the contacts to a stimulator unit. The conductive pathways may comprise any number of electrical conductors, such as a wire. FIG. 9A is a flowchart illustrating a method 900A of manufacturing a contact and conductive pathway arrangement of an electrode assembly in accordance with embodiments of the present invention. In the illustrative embodiments of FIG. 9A, method 900A begins at block 902 where an electrode contact is provided. Providing an electrode contact may comprise, for example, providing a contact which is pre-formed into a desired shape or configuration. In other embodiments, step 902 may further include forming an electrode contact into a desired shape or configuration. For example, in specific embodiments, providing an electrode contact includes providing an annular shaped contact, and forming the contact into a U-shape.

At block 904 a strain relief is formed in a conductive pathway. As discussed below, the strain relief may be formed in the conductive pathway using several methods. For example, a strain relief former tool in accordance with embodiments of the present invention may be used to form the strain relief. Also as described below, the strain relief may be formed at various different physical locations of the conductive pathway. As would be appreciated, the strain relief may be formed in the conductive pathway prior to the providing of an electrode contact. That is, in certain embodiments, step 904 may occur prior to, or concurrently with, step 902. As discussed below, in certain embodiments the strain relief is formed in the distal region of the conductive pathway.

At block 906, the distal end of the conductive pathway is attached to the electrode contact. As noted above, a variety of methods may be implemented to attach or join the distal end of the conductive pathway to the electrode contact. These methods may include, for example, resistance welding, wire bonding, crimping, laser welding, *etc.*

In the embodiments of FIG. 9A, the strain relief is formed in the conductive pathway prior to attaching the conductive pathway to an electrode contact. This helps to protect the contact joint from stresses that would be applied thereto if the strain relief was formed after the attachment process. Also, this facilitates automation of the manufacturing process because only wires, rather than wires attached to an electrode contact, must be manipulated to form the strain relief.

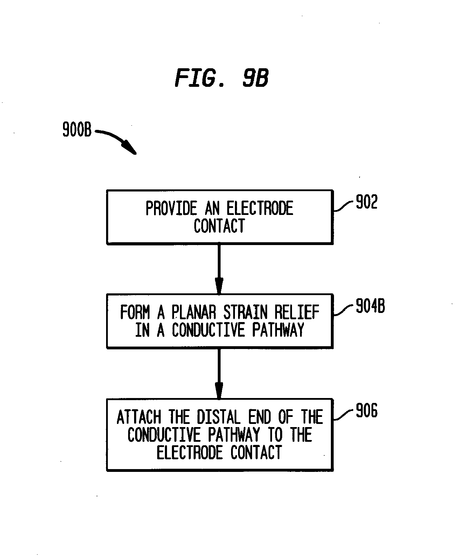

FIG. 9B is a flowchart illustrating a variation of method 900A, referred to as method 900B, for manufacturing a contact and wire arrangement of an electrode assembly in accordance with embodiments of the present invention. Similar to the embodiments of FIG. 9A, method 900B begins at block 902 where an electrode contact is provided. As noted, this step may include several variations, such as providing a pre-formed electrode contact, or forming a contact into a desired shape or configuration.

At block 904B a strain relief is formed in a conductive pathway. In the specific embodiments of FIG. 9B, step 904B includes the formation of a substantially planar strain relief. That is, the strain relief formed in step 904B is substantially positioned within a single plane. As discussed below, the planar strain relief may be formed, for example, by a strain relief former tool in accordance with embodiments of the present invention. Also as described below, the strain relief may be formed at various different locations of the conductive pathway. As would be appreciated, the strain relief may be formed in the conductive pathway prior to the providing of an electrode contact. That is, in certain embodiments, step 904B may occur prior to, or concurrently with, step 902.

At block 906, the distal end of the conductive pathway is attached to the electrode contact. As noted above, a variety of methods may be implemented to attach the distal end of the conductive pathway to the electrode contact, such as resistance welding, wire bonding, crimping, laser welding, *etc*.

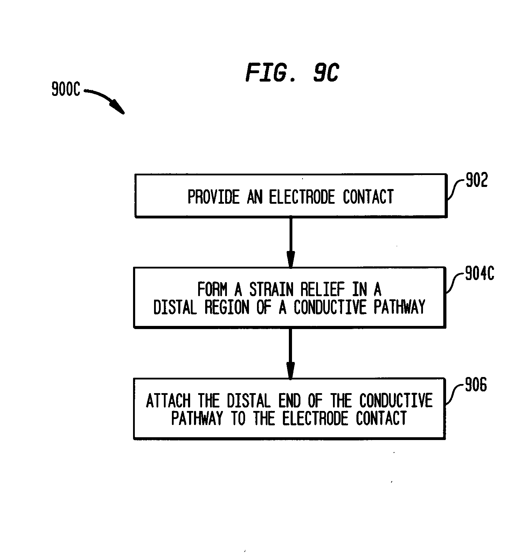

FIG. 9C is a flowchart illustrating another variation of method 900A, referred to as method 900C, for manufacturing a contact and wire arrangement of an electrode assembly in accordance with embodiments of the present invention. Similar to the embodiments of FIG. 9A, method 900C begins at block 902 where an electrode contact is provided. As noted, this step may include several variations, such as providing a pre-formed electrode contact, or forming a contact into a desired shape or configuration.

At block 904C a strain relief is formed in a conductive pathway. In the specific embodiments of FIG. 9C, step 904C includes the formation of a strain relief in a distal region of the conductive pathway. That is, the strain relief formed in step 904C is positioned at or near the distal end of the conductive pathway. As discussed below, the strain relief may be formed in the conductive pathway using several methods. For example, a strain relief former tool in accordance with embodiments of the present invention may be used to form the strain relief. As would be appreciated, the strain relief may be formed in the conductive pathway prior to the providing of an electrode contact. That is, in certain embodiments, step 904C may occur prior to, or concurrently with, step 902.

At block 906, the distal end of the conductive pathway is attached to the electrode contact. As noted above, a variety of methods may be implemented to attach the distal end of the conductive pathway to the electrode contact, such as resistance welding, wire bonding, crimping, laser welding, *etc.*

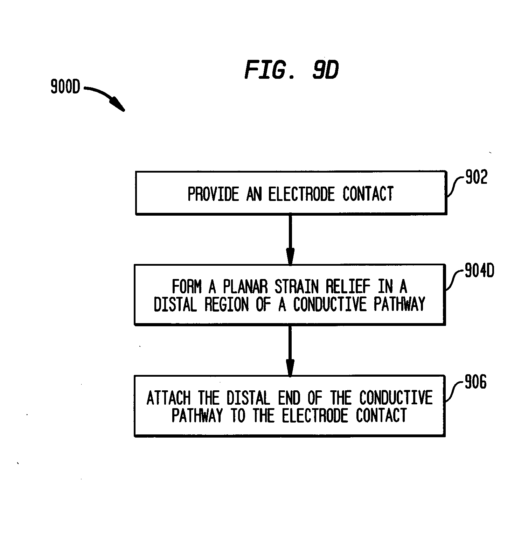

FIG. 9D is a flowchart illustrating a still other variation of method 900A, referred to as method 900D, for manufacturing a contact and wire arrangement of an electrode assembly in accordance with embodiments of the present invention. Similar to the embodiments of FIG. 9A, method 900D begins at block 902 where an electrode contact is provided. As noted, this step may include several variations, such as providing a pre-formed electrode contact, or forming a contact into a desired shape or configuration.

At block 904D a strain relief is formed in a conductive pathway. In the specific embodiments of FIG. 9D, step 904D includes the formation of a substantially planar strain relief in a distal region of the conductive pathway. That is, the strain relief formed in step 904D is substantially positioned within a single plane, and is positioned at or near the distal end of the conductive pathway. As discussed below, the planar strain relief may be formed, for example, by a strain relief former tool in accordance with embodiments of the present invention. As would be appreciated, the strain relief may be formed in the conductive pathway prior to the providing of an electrode contact. That is, in certain embodiments, step 904D may occur prior to, or concurrently with, step 902.

At block 906, the distal end of the conductive pathway is attached to the electrode contact. As noted above, a variety of methods may be implemented to attach the distal end of the conductive pathway to the electrode contact, such as resistance welding, wire bonding, crimping, laser welding, *etc.*

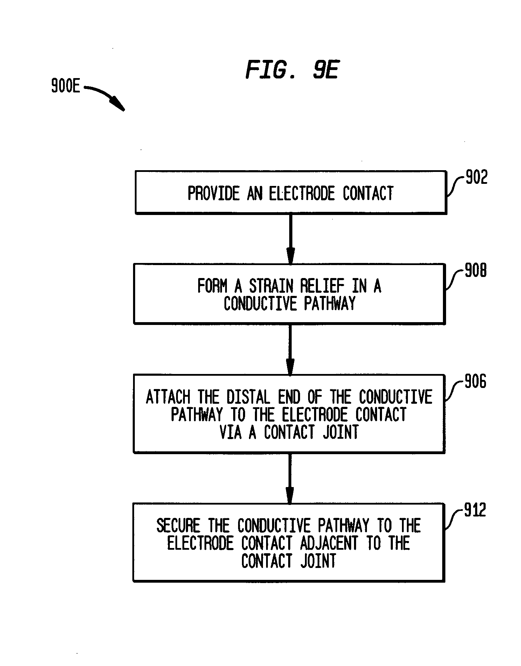

FIG. 9E is a flowchart illustrating a variation of method 900A, referred to as method 900E, for manufacturing a contact and wire arrangement of an electrode assembly in accordance with embodiments of the present invention. Similar to the embodiments of FIG. 9A, method 900E begins at block 902 where an electrode contact is provided. As noted, this step may include several variations, such as providing a pre-formed electrode contact, or forming a contact into a desired shape or configuration.

At block 908 a strain relief is formed in a conductive pathway using one of a variety of methods as described above with reference to FIG. 9A. As would be appreciated, the strain relief may be formed in the conductive pathway prior to the providing of an electrode contact. That is, in certain embodiments, step 908 may occur prior to, or concurrently with, step 902.

At block 906, the distal end of the conductive pathway is attached to the electrode contact via a contact joint. As noted above, a variety of methods may be implemented to attach the distal end of the conductive pathway to the electrode contact, such as resistance welding, wire bonding, crimping, laser welding, *etc.*

Method 900E includes the additional step at block 912 where the conductive pathway is secured to the electrode contact. Specifically, at block 912, a section of the conductive pathway substantially between the strain relief and the contact joint, and adjacent to the contact joint, is secured to the electrode contact. As described above with reference to FIGS. 8A-8C, several anchoring arrangements may be provided to secure or anchor the conductive pathway to the electrode contact.

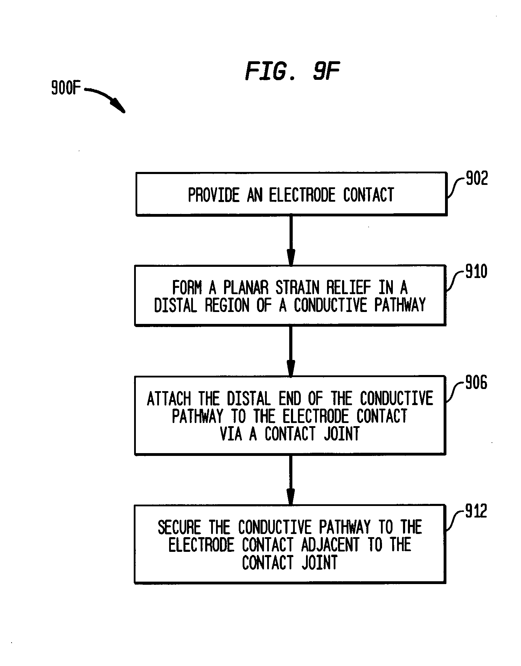

FIG. 9F is a flowchart illustrating another variation of method 900A, referred to as method 900F, for manufacturing a contact and wire arrangement of an electrode assembly in accordance with embodiments of the present invention. Similar to the embodiments of FIG. 9A, method 900F begins at block 902 where an electrode contact is provided. As noted, this step may include several variations, such as providing a pre-formed electrode contact, or forming a contact into a desired shape or configuration.

At block 910 a strain relief is formed in a conductive pathway. In the specific embodiments of FIG. 9F, step 910 includes the formation of a substantially planar strain relief in a distal region of the conductive pathway. That is, the strain relief formed in step 910 is substantially positioned within a single plane, and is positioned at or near the distal end of the conductive pathway. As discussed below, the planar strain relief may be formed, for example, by a strain relief former tool in accordance with embodiments of the present invention. As would be appreciated, the strain relief may be formed in the conductive pathway prior to the providing of an electrode contact. That is, in certain embodiments, step 910 may occur prior to, or concurrently with, step 902.

At block 906, the distal end of the conductive pathway is attached to the electrode contact via a contact joint. As noted above, a variety of methods may be implemented to attach the distal end of the conductive pathway to the electrode contact, such as resistance welding, wire bonding, crimping, laser welding, etc.

Method 900F includes the additional step at block 912 where the conductive pathway is secured to the electrode contact. Specifically, at block 912, a section of the conductive pathway substantially between the strain relief and the contact joint, and adjacent to the contact joint, the is secured to the contact. As described above with reference to FIGS. 8A-8C, several anchoring arrangements may be provided to secure or anchor the conductive pathway to the electrode contact.

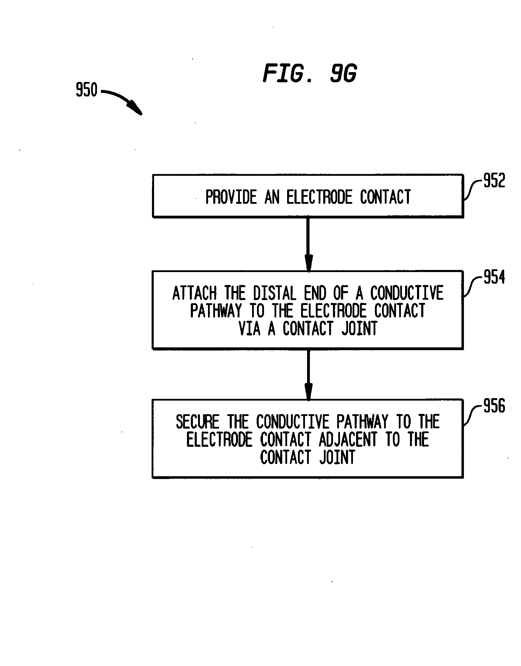

FIG. 9G is a flowchart illustrating a method 950 for manufacturing a contact and wire arrangement of an electrode assembly in accordance with embodiments of the present invention. Method 950 begins at block 952 where an electrode contact is provided. Similar to the embodiments described above, this step may include several variations, such as providing a pre-formed electrode contact, or forming a contact into a desired shape or configuration.

At block 954, the distal end of an elongate conductive pathway is attached to the electrode contact via a contact joint. Similar to the embodiments described above, a variety of methods may be implemented to attach the distal end of the conductive pathway to the electrode contact, such as resistance welding, wire bonding, crimping, laser welding, etc.

Method 950 includes the additional step at block 956 where the conductive pathway is secured to the electrode contact. Specifically, at block 956, a section of the conductive pathway adjacent to the contact joint is secured to the electrode contact. As described above with reference to FIGS. 8A-8C, several anchoring arrangements may be provided to secure or anchor the conductive pathway to the electrode contact.

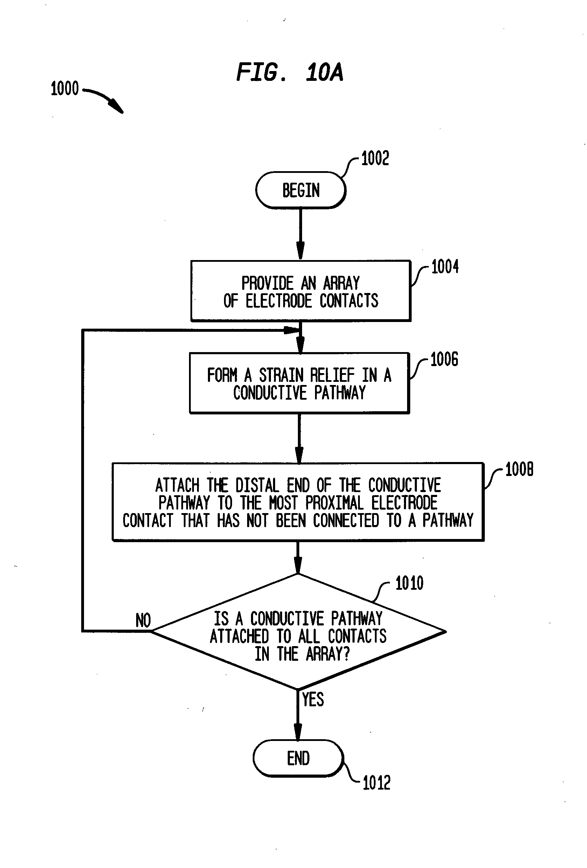

As noted, an electrode assembly in accordance with specific embodiments of the present invention includes several elements, such as a carrier member, a plurality of electrode contacts, and conductive pathways extending from each of the electrode contacts which electrically connect the contacts to a stimulator unit. FIG. 10A is a flowchart illustrating a method 1000 of manufacturing a contact and wire sub-assembly of an electrode assembly in accordance with embodiments of the present invention. In the illustrative embodiments of FIG. 10A, method 1000 begins at block 1002. A block 1004, an array of electrode contacts is provided. Providing an electrode array may comprise, for example, providing an array of contacts which are pre-formed into a desired shape, configuration or alignment. For example, in the embodiments of FIG. 10A, the contacts comprises an array of distally extending electrode contacts. In other embodiments, step 1004 may further include forming the electrode contacts within the array into a desired shape, configuration or alignment. For example, in specific embodiments, providing an array of electrode contacts includes providing annular shaped contacts, and forming the contacts into U-shapes.

At block 1006 a strain relief is formed in a conductive pathway. As discussed below, the strain relief may be formed in the conductive pathway using several methods. For example, a strain relief former tool in accordance with embodiments of the present invention may be used to form the strain relief. Also as described below, the strain relief may be formed at various different locations of the conductive pathway.

At block 1008, the distal end of the conductive pathway is attached to the most proximal electrode contact that has not yet been attached to a conductive pathway. As noted above, a variety of methods may be implemented to attach or join the distal end of the conductive pathway to the electrode contact. These methods may include, for example, resistance welding, wire bonding, crimping, laser welding, *etc.*

At block 1010, a determination is made as to whether a conductive pathway has been attached to all desired contacts in the electrode array. If a conductive pathway has been attached to all desired contacts within the array, the method ends at block 1012. However, if a conductive pathway has not been attached to all desired contacts, the method returns to block 1006 where a strain relief is formed in an additional conductive pathway. Steps 1006, 1008 and 1010 are repeated until a conductive pathway has been attached to all electrode contacts in the array.

In certain embodiments of the present invention, conductive pathways are attached to all contacts in the electrode array. In alternative embodiments, an electrode assembly may include non-active contacts, (ie contacts which are not connected to pathways, but are used to perform various other functions) as well as active contacts (i.e. contacts used to deliver electrical stimulation signals). In such embodiments, the conductive pathways are attached to only active contacts.

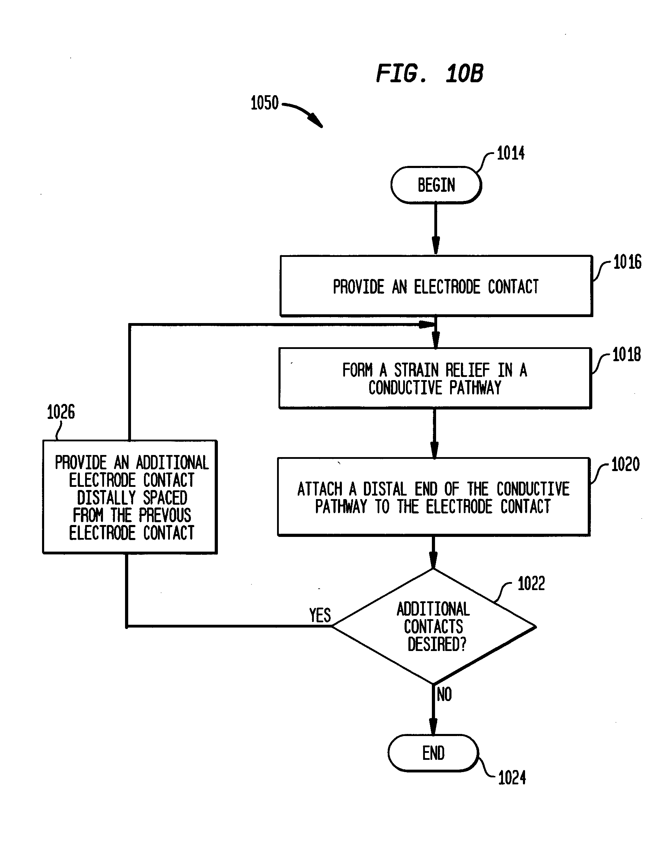

FIG. 10B is a flowchart illustrating an alternative method 1050 of manufacturing a contact and wire sub-assembly of an electrode assembly in accordance with embodiments of the present invention. In the illustrative embodiments of FIG. 10B, method 1050 begins at block 1014. A block 1016, an electrode contacts provided. Providing an electrode contact may comprise, for example, providing a contact that is pre-formed into a desired shape or configuration. In other embodiments, step 1016 may further include forming an electrode contact into a desired shape or configuration. For example, in specific embodiments, providing an electrode contact includes providing an annular shaped contact, and forming the contact into a U-shape.

At block 1018 a strain relief is formed in a conductive pathway. As discussed below, the strain relief may be formed in the conductive pathway using several methods. For example, a strain relief former tool in accordance with embodiments of the present invention may be used to form the strain relief. Also as described below, the strain relief may be formed at various different locations of the conductive pathway.

At block 1020, the distal end of the conductive pathway is attached to the provided electrode contact. As noted above, a variety of methods may be implemented to attach or join the distal end of the conductive pathway to the electrode contact. These methods may include, for example, resistance welding, wire bonding, crimping, laser welding, *etc.*

At block 1022, a determination is made as to whether additional contacts are desired. If additional contacts are not desired, the method ends at block 1024. However, if additional contacts are desired, the method proceeds to block 1026 where an additional contact is provided. In the specific embodiments of FIG. 10B, the additional electrode contact is distally spaced from the previous electrode contact. The method then returns to block 1018 where a strain relief is formed in an additional conductive pathway. Steps 1018, 1020, 1022 and 1026 are repeated until a conductive pathway has been attached to all electrode contacts in the array.

As noted above, in certain embodiments of the present invention, conductive pathways are attached to all contacts in the electrode array. In alternative embodiments, an electrode assembly may include non-active contacts, as well as active contacts. In such embodiments, an additional decision may be included to determine if a provided contact is designated as active or non-active. If a contact is non-active, a conductive pathway is not attached thereto, and the method proceeds to provide another contact.

In accordance with embodiments of the present invention, once an electrode array or contact and conductive pathway arrangement is manufactured using one of the methods of the present invention, an electrode assembly incorporating the component(s) may be completed. Forming an electrode assembly may include various steps, including following the welding of the pathways to the desired contacts, placing silicone in the troughs of each contact and placing a stylet (PTFE coated wire) on top of the conductive pathways and the silicone in the troughs of the contacts. This may further include partially filling each contact trough with additional silicone. The whole assembly is then placed in an oven to cure the silicone. The assembly is then curved and moulded in a curved moulding die.

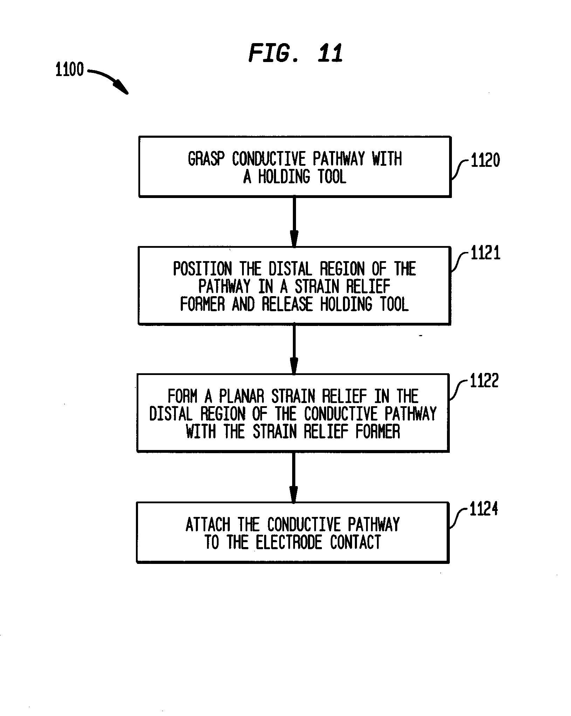

As noted above, embodiments of the present invention permit simple and automated manufacturing methods of small electrode assemblies that have decreased sensitivity to flexing/bending which may occur during normal use of the assembly. FIG. 11 is a flowchart illustrating a method 1100 of manufacturing a contact and conductive pathway arrangement of an electrode assembly in accordance with embodiments of the present invention which provides these and other advantages. Specifically, method 1100 is adapted to be partially or fully automated in embodiments of the present invention.

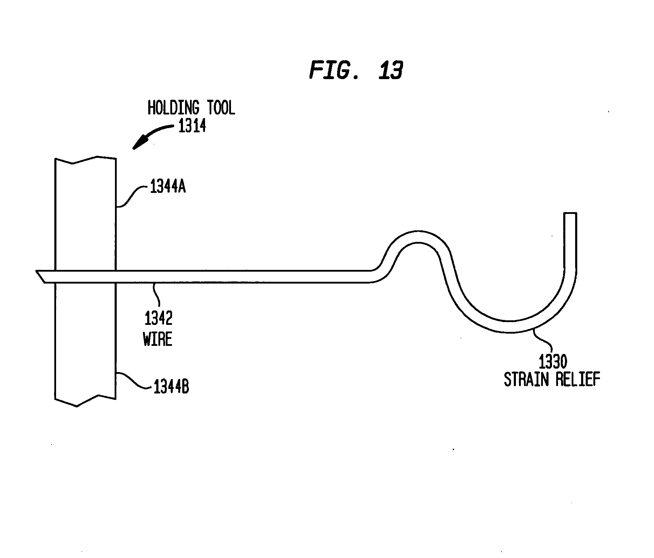

In the illustrative embodiments of FIG. 11, method 1100 begins at block 1120 where a holding tool is used to grasp an elongate conductive pathway. As shown in FIG. 13, the holding tool grasps the proximal end of the pathway. As described below with reference to FIG. 13, the holding tool may be a manual or machine operable tool.

In the embodiments of FIG. 11, the conductive pathway is a non-shaped relatively straight wire. As would be appreciated, a non-shaped wire is more readily held within an automated machine than a shaped wire. This results from the fact that a shaped wire requires very consistent shape and precise alignment of the holding tool to the shape. Because elongate wires are typically used to form electrode assembly, maintaining this precision required along the length of a shaped wire is extremely difficult.

At block 1121, the holding tool is used to position the conductive pathway within a strain relief former. Once positioned in the strain relief former, the conductive pathway is released by the holding tool. At block 1122, the strain relief former is used to form a planar strain relief in the distal region of the conductive pathway. As described below with reference to FIGS. 12A-12D, the holding tool may be a manual or machine operable tool. In the embodiments of FIG. 11, once the strain relief is formed, the distal end of the conductive pathway is positioned alongside the surface of an electrode contact. The distal end of the conductive pathway may then be attached to the electrode contact at block 1124.

As would be appreciated, strain reliefs that are in three dimensions may be difficult to form, thus making it difficult or impossible to automate the formation of such reliefs. As such, the manufacture of the wiring and connector components in which three dimensional reliefs are included is labor intensive and specialized manual craft. Also, three-dimensional strain reliefs must be rotated to confirm shape compliance further limiting manufacturing options.

As described above, in certain embodiments of the present invention, the strain relief is optimized to facilitate the automated forming of the strain relief. Automation of the strain relief formation has significant advantages in the form of reduced costs and the formation of consistent strain reliefs that are do not vary as a result of human errors. For example, in certain embodiments, the strain relief comprises a planar strain relief that is in two dimensions. Because the relief is only within a two-dimensional plane, the formation of the strain relief is less complicated than a three-dimensional relief, thereby facilitating automation of the strain relief formation.

Furthermore, as described below, in embodiments of the present invention, the physical shape of the relief is selected to facilitate the automated formation of the strain relief. For example, in certain embodiments, a strain relief comprises multiple planar turns or bends of wire. The turns are largest at the most distal end, and decrease in size towards the proximal end of the relief. This decreasing shape of the turns facilitates the automation process. Other alterations to the shape of the strain relief to facilitate automation are within the scope of the present invention.

Partial or full automation of the process used to manufacture any elements of an electrode assembly, such as a contact or conductive pathway arrangement, has numerous advantages. FIGS. 12A and 12B are perspective views of a strain relief forming tool 1200A in accordance with embodiments of the present invention that may facilitate automation of the manufacturing process. Strain relief former 1200A is used to form a planar strain relief in a distal region of a conductive pathway. As shown, strain relief former 1200A comprises a first portion 1202, and an opposing second portion 1204. First portion 1202 comprises a curved surface 1224 that is configured to approach a mating surface 1226 in second portion 1204.

In operation, a wire 1242 is placed between first and second portions 1202, 1204. The first and second portions are then closed so that surfaces 1224, 1226 approach one another. As surfaces 1224, 1226 approach one another, a non-linear section of wire 1242 is formed, referred to as strain relief 1230. Furthermore, in the embodiments of FIG. 12B, as portions 1202, 1204 approach one another, surfaces 1220, 1222 cut wire 1242 to the desired length immediately before strain relief 1230 is formed.

Strain relief 1230 formed by strain relief former 1200A comprises a non-linear section of wire 1242. In particular, strain relief 1230 comprises a full turn 1211 of wire 1242, and a half turn 1213 of the wire. As noted above, a full turn of wire or conductive pathway refers to a section of wire/pathway that travels a first direction for a first distance, has a general U-shaped portion, and which extends away from the U-shape for a second distance which is approximately the same as the first distance. In contrast, a half turn refers to a section of wire that only comprises a U-shaped potion.

As noted above, in embodiments of the present invention the turns of a strain relief, such as strain 1230, are asymmetric in size. In some embodiments, each distally positioned turn is larger than the next most proximal turn. In other embodiments, each distally positioned turn is smaller than the next most proximal turn. The size of a turn refers to the change in length of wire over the original longitudinal distance of the wire. It should be appreciated that an increase in size increases the largest dimensions of the turn perpendicular to the longitudinal axis of wire 1242.

FIGS. 12A-12D illustrate embodiments in which the turns of strain relief 1230 decrease in size from the distal end, and are referred to herein as decreasing turns of wire 1242. That is, turn 1213 is smaller in size than turn 1211 that is closest to the distal end of wire 1242.

As noted, the asymmetrical size of turns 1211 and 1213 is important to facilitate the automated formation of strain relief 1230 by permitting the formation to occur by a single action tool. Specifically, as shown in FIGS. 12A and 12B, when surfaces 1224 and 1226 having members 1201, 1205, and 1203, and 1207, respectively, approach one another, member 1201 will contact wire 1242 before member 1207 contacts the wire. As such, a substantial portion of turn 1211 is formed prior to the formation of turn 1213.

If turns 1211 and 1213 were the same size, members 1201 and 1207 would contact the wire at the same time. This would pull wire 1242 at two locations simultaneously and place a tension on wire 1242 that would likely break to damage wire. In contrast, the delay in the formation of turn 1213 provides the ability to form multiple turns without placing the undesired tension on wire 1242.

As would be appreciated, in order to form multiple turns of wire having the same size, a multi-stage formation tool would be required. In particular, a different tool would be needed to form each turn in the relief so as to prevent breakage of the wire due to tensile forces. As such, the wire must be moved from tool to tool to complete the procedure. As would be appreciated, the need to realign the wire within each tool is disadvantageous. As noted, embodiments of the present invention avoid the need for a multistage tool and form multiple turns with a single action tool.

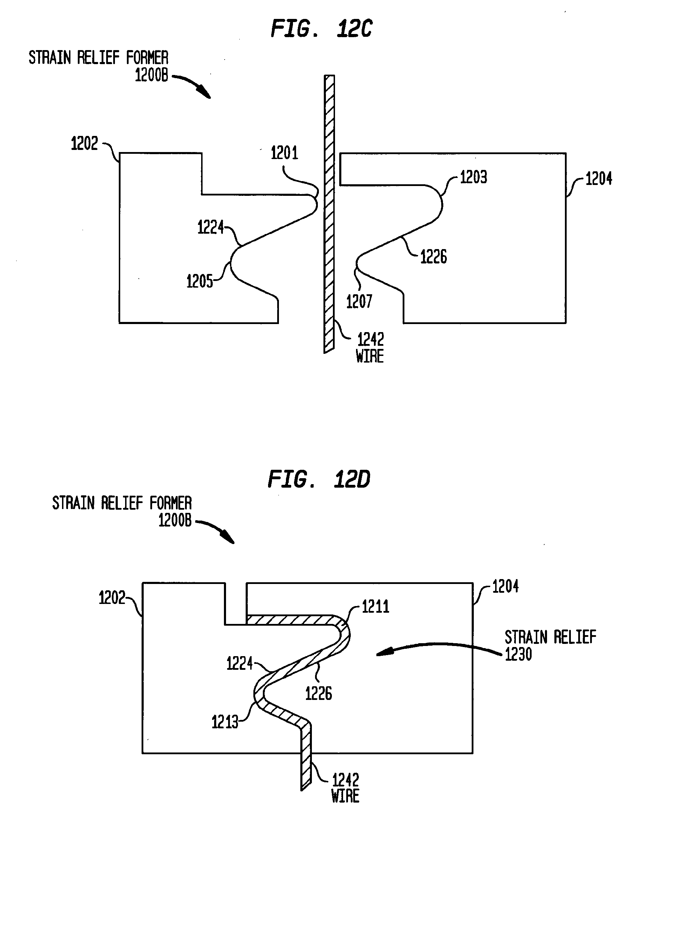

FIGS. 12C and 12D are perspective views of an alternative strain relief former 1200B that may be used to form a strain relief in embodiments of the present invention. Strain relief former 1200B is substantially similar to strain relief former 1200A of FIGS. 12A and 12B. However, in the illustrative embodiments of FIGS. 12A and 12B, portions 1202, 1204 do not include surfaces 1220, 1222 that cut wire 1242 to the desired length immediately before forming strain relief 1230. As such, in these embodiments, wire 1242 would be cut to the desired length prior to positioning the wire in the strain relief former, or after removal of wire 1242 from the strain relief former.

As noted above with reference to FIGS. 12A and 12B, the turns of strain relief 1230 decrease in size from the distal end, and are referred to herein as decreasing turns of wire 1242. That is, turn 1213 is smaller in size than turn 1211 that is closest to the distal end of wire 1242. The size of a turn refers to the largest dimensions of the turn perpendicular to the longitudinal axis of wire 1242.

As noted, the embodiments of FIGS. 12A-12D illustrate a strain relief 1230 comprising a full turn 1211, and a half turn 1213. As would be appreciated, in alternative embodiments, turn 1213 may comprise a full turn and/or additional turns may be added to strain relief 1230. In such embodiments, the turns are asymmetrical in size.

The longer the stored length of the wire within strain relief, the greater the change in length allowed and the more effective the stain relief is. However, the bend radius of a wire is limited, thus limiting the amount of wire that may be stored for use as the strain relief. Bend radius refers to the fact that when a wire is bent, the outside of the bend is under tensile stress and the inside under compressive stress. If the bend radius is too small then weaknesses occur in the bend zone. Thus, the bend radius is limited to a ratio of the thickness of the wire. For example, a wire used in certain embodiments of the present invention comprises a Pt/Ir wire, and has a diameter of 25 microns having a bend radius of approximately 100microns. As would be appreciated, changing the temper of the wire (e.g. annealing) would affect the acceptable bend radius and the properties of the strain relief. Furthermore, wires having different properties such as different cross sections (eg rectangular for 'machined foil strips') or different materials / alloy combinations (eg using Pt not Pt/Ir) would also have different bend radius limitation. Therefore, strain former tools 1200A, 1200B are configured to place bends in wire 1242 are within bend radius limitations.

As noted above in FIG. 11, in certain embodiments of the present invention, a holding tool may be used to grasp a conductive pathway to, for example, position the conductive pathway in a strain relief former. FIG. 13 is a perspective view of holding tool 1314, in accordance with embodiments of the present invention used to grasp an elongate wire 1342. As shown, holding tool 1314 comprises opposing members 1344 that grasp and hold the proximal end of wire 1342. In accordance with embodiments of the present invention, holding tool 1314 comprises an air cylinder with holding features (1344) are customized for holding and grasping a conductive pathway. Furthermore, the mating surfaces of opposing members 1344 are lined with a layer of rubber (e.g. Viton fluoroelastomer, silicone, *etc*.)

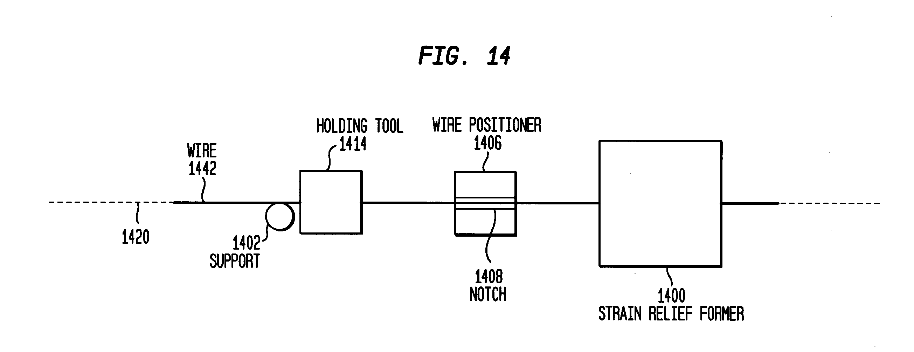

FIG. 14 is a block diagram illustrating the use of a holding tool and strain relief former in accordance with embodiments of the present invention. FIG. 14 illustrates the side view of a support 1402, a holding tool 1414, a wire guide 1410, a wire positioned 1406 and a strain relief former 1400. Strain relief former 1400 and holding tool 1414 may be implemented as described above with reference to FIGS. 12A-12D and FIG. 13, respectively, and not described further herein. As such, because holding tool 1414 and strain relief former 1400 may have several different arrangements, the holding tool and strain relief former are schematically illustrated as boxes.

As shown, wire 1442 is aligned with an axis 1420. For ease of illustration, axis 1420 is shown as a horizontal axis. However, it would be appreciated that axis 1420 may be disposed at a number of angles. For example, in certain embodiments, axis 1420 may be disposed at an angle of approximately 15 degrees from a horizontal axis.

In the embodiments of FIG. 14, wire 1442 extends first to a support 1402 that may comprise, for example, a stainless steel pin. Wire 1442, supported by pin 1402, extends through holding tool 1414. As noted above, holding tool 1414 comprises opposing members that grasp and hold wire 1442. From holding tool 1414, wire 1442 extends through a v-notch 1408 of wire positioner 1406 to strain relief former 1400 for formation of a strain relief as described above with reference to FIGS 12A-12D.

During the formation of a strain relief in accordance with certain embodiments of the present invention, a wire is secured at both sides of a strain relief former. However, in other embodiments of the present invention, the wire may be secured only at one side of the former, or alternatively, at neither side of the former.

## Claims

1. An implantable electrode assembly (218) comprising:
a carrier member;
at least one electrode contact (248, 548, 648, 748, 848) disposed in the carrier member;
at least one elongate conductive pathway (342) disposed in the carrier member having a distal end attached to the at least one electrode contact and, **characterized by** the at least one elongate conductive pathway having a substantially planar strain relief (330, 430, 530, 630, 730, 830) formed therein that is located only in the distal region of the pathway (342, 442),
wherein the carrier member is an elongate carrier member.

2. The electrode assembly (218) of claim 1, wherein the strain relief (330, 430, 530, 630, 730, 830) is positioned alongside the at least one electrode contact (248, 548, 648, 748, 848), and wherein the strain relief (330, 430, 530, 630, 730, 830) has a length that is less than the length of the at least one electrode contact (248, 548, 648, 748, 848).

3. The electrode assembly (218) of any of the proceeding claims, wherein the at least one electrode contact (248, 548, 648, 748, 848) comprises a plurality of distally extending electrode contacts separated by electrode gaps, and wherein the strain relief (330, 430, 530, 630, 730, 830) has a length that is less than the combined length of the electrode contact (248, 548, 648, 748, 848) to which the distal end of the pathway (342) is attached and an electrode gap (340).

4. The electrode assembly (218) of any of the proceeding claims, wherein an elongate portion of the electrode assembly (218) is configured to be implanted in a recipient's cochlea (140), and wherein the strain relief (330, 430, 530, 630, 730, 830) has a length that is approximately the same as the distance between one of the proximal and distal end of the at least one electrode contact (248, 548, 648, 748, 848) and the proximal end of the portion of the electrode assembly (218) implanted in the cochlea (140).

5. The electrode assembly (218) of any of the proceeding claims, wherein the strain relief (330, 430, 530, 630, 730, 830) comprises a section of the at least one pathway (342) formed into at least one and a half turns.

6. The electrode assembly (218) of any of the proceeding claims, wherein the strain relief (330, 430, 530, 630, 730, 830) comprises a section of the at least one pathway (342) formed into a substantially annular shape.

7. The electrode assembly (218) of any of the proceeding claims, wherein the strain relief (330, 430, 530, 630, 730, 830) comprises a section of the at least one pathway (342) formed into a plurality of annular coils.

8. The electrode assembly (218) of any of the proceeding claims, wherein the distal end of the at least one pathway (342) is attached to the at least one electrode contact (248, 548, 648, 748, 848) via a contact joint (444, 544, 744, 844), and wherein the electrode assembly (218) further comprises:
an anchor arrangement (820, 824) securing the at least one pathway (342) to the at least one electrode contact (248, 548, 648, 748, 848) adjacent to the contact joint (444, 544, 744, 844).

9. The electrode assembly (218) of any of the proceeding claims, wherein the anchor arrangement (820, 824) comprises:
a section of the at least one pathway (342) adjacent to the contact joint (444, 544, 744, 844) positioned in close proximity to the at least one electrode contact (248, 548, 648, 748, 848); and
a material disposed between the section of the at least one pathway (342) and the at least one electrode contact (248, 548, 648, 748, 848) configured to adhere the pathway (342) to the electrode contact (248, 548, 648, 748, 848).

10. The electrode assembly (218) of any of the proceeding claims, wherein the material comprises at least one of silicone, silicone adhesive, silicone rubber and medical grade epoxy.

11. The electrode assembly (218) of any of the proceeding claims, wherein the anchor arrangement (820, 824) comprises a section of the at least one pathway (342) between the strain relief (330, 430, 530, 630, 730, 830) and the contact joint (444, 544, 744, 844) welded to the at least one electrode contact (248, 548, 648, 748, 848).

12. The electrode assembly (218) of any of the proceeding claims, wherein the at least one electrode contact (248, 548, 648, 748, 848) has a post (838) extending from the surface thereof, and wherein the anchor arrangement (820, 824) comprises a section of the at least one pathway (342) between the strain relief (330, 430, 530, 630, 730, 830) and the contact joint (444, 544, 744, 844) at least partially wound around the post (838).

13. The electrode assembly (218) of any of the proceeding claims, wherein the at least one electrode contact (248, 548, 648, 748, 848) comprises a plurality of distally extending electrode contacts (248, 548, 648, 748, 848), and wherein the assembly (218) further comprises:
a plurality of elongate conductive pathways disposed in the carrier member each having a distal end attached to one of said plurality of electrode contacts (248, 548, 648, 748, 848) and each having a substantially planar strain relief (330, 430, 530, 630, 730, 830) formed therein that is positioned only proximate to the respective electrode contact (248, 548, 648, 748, 848) to which the pathway is attached.

14. The electrode assembly (218) of any of the proceeding claims, wherein a strain relief (330, 430, 530, 630, 730, 830) formed in a first one of the plurality of pathways has one or more of a different shape and width than a strain relief formed in a second pathway.

## Patentansprüche

1. Implantierbare Elektrodenanordnung (218), die Folgendes umfasst:
ein Trägerelement;
mindestens einen Elektrodenkontakt (248, 548, 648, 748, 848), der auf dem Trägerelement angeordnet ist;
mindestens eine langgestreckte leitfähige Leitung (342), die auf dem Trägerelement angeordnet ist, mit einem distalen Ende, das an dem mindestens einen Elektrodenkontakt angefügt ist, und
**dadurch gekennzeichnet, dass**
die mindestens eine langgestreckte leitfähige Leitung eine im Wesentlichen planare mechanische Spannungsentlastung (330, 430, 530, 630, 730, 830) aufweist, die darin ausgebildet ist, die nur im distalen Bereich der Leitung (342, 442) angeordnet ist,
wobei das Trägerelement ein lang gestrecktes Trägerelement ist.

2. Elektrodenanordnung (218) nach Anspruch 1, wobei die mechanische Spannungsentlastung (330, 430, 530, 630, 730, 830) längsseits des mindestens einen Elektrodenkontakts (248, 548, 648, 748, 848) positioniert ist, und wobei die mechanische Spannungsentlastung (330, 430, 530, 630, 730, 830) eine Länge aufweist, die kleiner ist als die Länge des mindestens einen Elektrodenkontakts (248, 548, 648, 748, 848).

3. Elektrodenanordnung (218) nach einem der vorhergehenden Ansprüche, wobei der mindestens eine Elektrodenkontakt (248, 548, 648, 748, 848) eine Vielzahl von sich distal erstreckenden Elektrodenkontakten umfasst, die durch Elektrodenlücken voneinander getrennt sind, und wobei die mechanische Spannungsentlastung (330, 430, 530, 630, 730, 830) eine Länge aufweist, die kleiner ist als die kombinierte Länge des Elektrodenkontakts (248, 548, 648, 748, 848), an dem das distale Ende der Leitung (342) angebracht ist, und einer Elektrodenlücke (340).

4. Elektrodenanordnung (218) nach irgendeinem der vorhergehenden Ansprüche, wobei der langgestreckte Bereich der Elektrodenanordnung (218) konfiguriert ist, so dass er in einer Gehörschnecke (140) eines Empfängers implantiert werden kann, und wobei die mechanische Spannungsentlastung (330, 430, 530, 630, 730, 830) eine Länge aufweist, die ungefähr genauso groß ist wie der Abstand zwischen entweder dem proximalen oder dem distalen Ende des mindestens einen Elektrodenkontakts (248, 548, 648, 748, 848) und dem proximalen Ende des Bereichs der Elektrodenanordnung (218), der in der Gehörschnecke (140) implantiert ist.

5. Elektrodenanordnung (218) nach irgendeinem der vorangegangenen Ansprüche, wobei die mechanische Spannungsentlastung (330, 430, 530, 630, 730, 830) einen Abschnitt der mindestens einen Leitung (342) umfasst, der als mindestens ein und eine halbe Windung ausgebildet ist.

6. Elektrodenanordnung (218) nach irgendeinem der vorangegangenen Ansprüche, wobei die mechanische Spannungsentlastung (330, 430, 530, 630, 730, 830) einen Abschnitt der mindestens einen Leitung (342) umfasst, der als eine im Wesentlichen ringförmige Form ausgebildet ist.

7. Elektrodenanordnung (218) nach einem der vorangegangenen Ansprüche, wobei die mechanische Spannungsentlastung (330, 430, 530, 630, 730, 830) einen Abschnitt der mindestens einen Leitung (342) umfasst, die als eine Vielzahl von kreisförmigen Windungen ausgebildet sind.

8. Elektrodenanordnung (218) nach einem der vorangegangenen Ansprüche, wobei das distale Ende der mindestens einen Leitung (342) an dem mindestens einen Elektrodenkontakt (248, 548, 648, 748, 848) über eine Kontaktverbindung (444, 544, 744, 844) angebracht ist, und wobei die Elektrodenanordnung (218) weiterhin umfasst:
eine Ankeranordnung (820, 824), die die mindestens eine Leitung (342) an dem mindestens einen Elektrodenkontakt (248, 548, 648, 748, 848) neben der Kontaktverbindung (444, 544, 744, 844) fixiert.

9. Elektrodenanordnung (218) nach irgendeinem der vorangegangenen Ansprüche, wobei die Ankeranordnung (820, 824) Folgendes umfasst:
einen Abschnitt der mindestens einen Leitung (342) angrenzend an die Kontaktverbindung (444, 544, 744, 844), der in der nahen Umgebung des mindestens einen Elektrodenkontakts (248, 548, 648, 748, 848) positioniert ist; und
ein Material, das zwischen dem Bereich der mindestens einen Leitung (342) und dem mindestens einen Elektrodenkontakt (248, 548, 648, 748, 848) angeordnet ist, das konfiguriert ist, die Leitung (342) an den Elektrodenkontakt (248, 548, 648, 748, 848) zu heften.

10. Elektrodenanordnung (218) nach irgendeinem der vorangegangenen Ansprüche, wobei das Material mindestens eins der folgenden Materialien umfasst: Silicon, Siliconkleber, Silicongummi und Epoxid mit medizinischer Güteklasse.

11. Elektrodenanordnung (218) nach irgendeinem der vorhergehenden Ansprüche, wobei die Ankeranordnung (820, 824) einen Abschnitt der mindestens einen Leitung (342) zwischen der mechanische Spannungsentlastung (330, 430, 530, 630, 730, 830) und der Kontaktverbindung (444, 544, 744, 844) umfasst, der an den mindestens einen Elektrodenkontakt (248, 548, 648, 748, 848) geschweißt ist.

12. Elektrodenanordnung (218) nach einem der vorangegangenen Ansprüche, wobei der mindestens Elektrodenkontakt (248, 548, 648, 748, 848) einen Pfosten (838) aufweist, der sich von dessen Oberfläche heraus erstreckt, und wobei die Ankeranordnung (820, 824) einen Abschnitt der mindestens einen Leitung (342) zwischen der mechanische Spannungsentlastung (330, 430, 530, 630, 730, 830) und der Kontaktverbindung (444, 544, 744, 844) umfasst, der zumindest teilweise um den Pfosten (838) gewunden ist.

13. Elektrodenanordnung (218) nach irgendeinem der vorangegangenen Ansprüche, wobei der mindestens eine Elektrodenkontakt (248, 548, 648, 748, 848) eine Vielzahl von sich distal heraus erstreckende Elektrodenkontakte (248, 548, 648, 748, 848) umfasst, und wobei die Anordnung (218) weiterhin umfasst:
eine Vielzahl von langgestreckten leitfähigen Leitungen, die in dem Trägerelement angeordnet sind, die jeweils ein distales Ende aufweisen, die an einem der Vielzahl von Elektrodenkontakten (248, 548, 648, 748, 848) angebracht sind und die jeweils eine im Wesentlichen planare mechanische Spannungsentlastung (330, 430, 530, 630, 730, 830), die darin ausgebildet sind, aufweist, die nur benachbart zu dem entsprechenden Elektrodenkontakt (248, 548, 648, 748, 848), an den die Leitung angebracht ist, positioniert ist.

14. Elektrodenanordnung (218) nach einem der vorangegangenen Ansprüche, wobei eine mechanische Spannungsentlastung (330, 430, 530, 630, 730, 830), die in einer ersten einer Vielzahl von Leitungen ausgebildet ist, eine oder mehrere Formen und Breiten aufweist, die unterschiedlich sind zu einer mechanische Spannungsentlastung, die in einer zweiten Leitung ausgebildet ist.

## Revendications

1. Ensemble d'électrode implantable (218), comprenant :
un élément porteur ;
au moins un contact d'électrode (248, 548, 648, 748, 848) disposé dans l'élément porteur ;
au moins un chemin conducteur allongé (342) disposé dans l'élément porteur et ayant une extrémité distale attachée audit au moins un contact d'électrode, et
**caractérisé en ce que** ledit au moins un chemin conducteur allongé comporte un réducteur de contrainte essentiellement plat (330, 430, 530, 630, 730, 830) formé dans celui-ci et uniquement situé dans la région distale du chemin (342, 442),
dans lequel l'élément porteur est un élément porteur allongé.

2. Ensemble d'électrode (218) selon la revendication 1, dans lequel le réducteur de contrainte (330, 430, 530, 630, 730, 830) est positionné le long dudit au moins un contact d'électrode (248, 548, 648, 748, 848), et dans lequel le réducteur de contrainte (330, 430, 530, 630, 730, 830) présente une longueur inférieure à la longueur dudit au moins un contact d'électrode (248, 548, 648, 748, 848).

3. Ensemble d'électrode (218) selon l'une quelconque des revendications précédentes, dans lequel ledit au moins un contact d'électrode (248, 548, 648, 748, 848) comprend une pluralité de contacts d'électrode qui s'étendent distalement, séparés par des intervalles d'électrode, et dans lequel le réducteur de contrainte (330, 430, 530, 630, 730, 830) présente une longueur inférieure à la longueur combinée du contact d'électrode (248, 548, 648, 748, 848) auquel l'extrémité distale du chemin (342) est attachée et d'un intervalle d'électrode (340).

4. Ensemble d'électrode (218) selon l'une quelconque des revendications précédentes, dans lequel une partie allongée de l'ensemble d'électrode (218) est configurée pour être implantée dans la cochlée d'un receveur (140), et dans lequel le réducteur de contrainte (330, 430, 530, 630, 730, 830) présente une longueur approximativement égale à la distance entre une extrémité parmi l'extrémité proximale et l'extrémité distale dudit au moins un contact d'électrode (248, 548, 648, 748, 848) et l'extrémité proximale de la partie de l'ensemble d'électrode (218) implantée dans la cochlée (140).

5. Ensemble d'électrode (218) selon l'une quelconque des revendications précédentes, dans lequel le réducteur de contrainte (330, 430, 530, 630, 730, 830) comprend une section dudit au moins un chemin (342) formant au moins un tour et demi.

6. Ensemble d'électrode (218) selon l'une quelconque des revendications précédentes, dans lequel le réducteur de contrainte (330, 430, 530, 630, 730, 830) comprend une section dudit au moins un chemin (342) de forme sensiblement annulaire.

7. Ensemble d'électrode (218) selon l'une quelconque des revendications précédentes, dans lequel le réducteur de contrainte (330, 430, 530, 630, 730, 830) comprend une section dudit au moins un chemin (342) formant une pluralité de spires annulaires.

8. Ensemble d'électrode (218) selon l'une quelconque des revendications précédentes, dans lequel l'extrémité distale dudit au moins un chemin (342) est attachée audit au moins un contact d'électrode (248, 548, 648, 748, 848) via une jointure de contact (444, 544, 744, 844), et dans lequel l'ensemble d'électrode (218) comprend en outre :
un dispositif d'ancrage (820, 824) fixant ledit au moins un chemin (342) audit au moins un contact d'électrode (248, 548, 648, 748, 848) en un point adjacent à la jointure de contact (444, 544, 744, 844).

9. Ensemble d'électrode (218) selon l'une quelconque des revendications précédentes, dans lequel le dispositif d'ancrage (820, 824) comprend :
une section dudit au moins un chemin (342) adjacente à la jointure de contact (444, 544, 744, 844) et positionnée à forte proximité dudit au moins un contact d'électrode (248, 548, 648, 748, 848) ; et
un matériau disposé entre la section dudit au moins un chemin (342) et ledit au moins un contact d'électrode (248, 548, 648, 748, 848), configuré pour coller le chemin (342) au contact d'électrode (248, 548, 648, 748, 848).

10. Ensemble d'électrode (218) selon l'une quelconque des revendications précédentes, dans lequel le matériau comprend au moins un composant parmi le silicone, un adhésif au silicone, un caoutchouc au silicone, et un polyépoxyde pour applications médicales.

11. Ensemble d'électrode (218) selon l'une quelconque des revendications précédentes, dans lequel le dispositif d'ancrage (820, 824) comprend une section dudit au moins un chemin (342) entre le réducteur de contrainte (330, 430, 530, 630, 730, 830) et la jointure de contact (444, 544, 744, 844) soudée audit au moins un contact d'électrode (248, 548, 648, 748, 848).

12. Ensemble d'électrode (218) selon l'une quelconque des revendications précédentes, dans lequel ledit au moins un contact d'électrode (248, 548, 648, 748, 848) comporte un ergot (838) qui s'étend depuis sa surface, et dans lequel le dispositif d'ancrage (820, 824) comprend une section dudit au moins un chemin (342) entre le réducteur de contrainte (330, 430, 530, 630, 730, 830) et la jointure de contact (444, 544, 744, 844) au moins partiellement enroulée autour de l'ergot (838).

13. Ensemble d'électrode (218) selon l'une quelconque des revendications précédentes, dans lequel ledit au moins un contact d'électrode (248, 548, 648, 748, 848) comprend une pluralité de contacts d'électrode qui s'étendent distalement (248, 548, 648, 748, 848), et dans lequel l'ensemble (218) comprend en outre :
une pluralité de chemins conducteurs allongés disposés dans l'élément porteur, ayant chacun une extrémité distale attachée à un contact d'électrode de ladite pluralité de contacts d'électrode (248, 548, 648, 748, 848), et ayant chacun un réducteur de contrainte essentiellement plat (330, 430, 530, 630, 730, 830) qui y est formé et est positionné uniquement à proximité du contact d'électrode respectif (248, 548, 648, 748, 848) auquel le chemin est attaché.

14. Ensemble d'électrode (218) selon l'une quelconque des revendications précédentes, dans lequel un réducteur de contrainte (330, 430, 530, 630, 730, 830) formé dans un premier chemin de la pluralité de chemins présente une ou plusieurs différences, parmi la forme et la largeur, par rapport à un réducteur de contrainte formé dans un deuxième chemin.
